# DEMANDE DE BREVET EUROPEEN

(11) **EP 4 710 960 A1**
(43) Date de publication de la demande: **18.03.2026**
(21) Numéro de dépôt: 25201245.5
(22) Date de dépôt: 09.09.2025
(51) Int. Cl.: A61L 27/36, A61F 2/82, A61F 9/00, A61K 35/50, A61L 27/50

(54) **DISPOSITIF IMPLANTABLE CONSTITUÉ DE PAROI DE CORDON OMBILICAL**

(30) Priorité: 11.09.2024 FR 2409669
(71) Demandeur: TBF Genie Tissulaire (TBF), 69780 Mions (FR)
(72) Inventeur: BARNOUIN, Laurence, 69780 MIONS (FR)
(74) Mandataire: Tripoz, Inès

(57) **Abrégé**

La présente invention concerne le domaine des dispositifs implantables utilisable en tant qu'allogreffe en chirurgie.

Elle concerne en particulier un procédé de fabrication d'un dispositif implantable consistant en une paroi de cordon ombilical réticulée, ledit dispositif implantable pouvant être choisi dans le groupe comprenant une lentille biologique, un implant intra-cornéen consistant en un anneau ou un segment anneau, un guide de régénération nerveuse, ligamentaire et/ou tendineuse, et/ou une valve cardiaque artificielle.

L'invention concerne également les dispositifs ainsi obtenus et leurs utilisations chirurgicales.

## Description

La présente invention concerne le domaine des dispositifs implantables utilisables en tant qu'allogreffe en chirurgie.

Dans le cadre de l'évolution constante des techniques de greffe, l'utilisation de dispositifs implantables issus de tissus biologiques comme allogreffes en chirurgie présente des avantages significatifs, notamment en termes de biocompatibilité et de régénération tissulaire.

Cependant, la fabrication de ces dispositifs représente un véritable défi. En effet, il est particulièrement difficile de reproduire des formes et des structures aussi précises et régulières que celles obtenues à partir de matériaux composites synthétiques.

De plus, les processus de traitement et de conservation des tissus biologiques posent des défis supplémentaires, rendant la production de dispositifs fiables et uniformes encore plus complexe.

Un premier défi réside dans l'identification du tissu biologique de départ à utiliser car de nombreuses caractéristiques essentielles des dispositifs implantables finaux, telles que la résistance, la taille et en particulier l'épaisseur, sont conditionnées par la nature même de ce tissu. De plus, ce tissu doit être disponible en quantité suffisante pour permettre son utilisation à grande échelle, tout en respectant les normes de bioéthique en vigueur.

Ces difficultés sont exacerbées par les problèmes de compatibilité et de tolérance immunologique, qui peuvent entraîner des réactions de rejet ou une dégradation prématurée du tissu implanté.

Un autre défi majeur réside dans la préparation du tissu biologique. Ce processus doit avant tout réduire les probabilités de rejet du greffon en minimisant les réactions immunitaires potentielles. Parallèlement, la préparation doit permettre la mise en forme précise du dispositif, afin qu'il puisse être utilisé directement et facilement par le chirurgien lors de l'intervention. Cela inclut la nécessité de maintenir l'intégrité structurale et fonctionnelle du tissu tout en le modifiant pour répondre aux exigences spécifiques de l'application chirurgicale, ce qui représente un équilibre délicat à atteindre.

Ainsi, la présente demande vise à la mise au point de dispositifs implantables utilisables en tant qu'allogreffes en chirurgie, en particulier en tant que lentille biologique, implant intra-cornéen consistant en un anneau ou un segment d'anneau, guide de régénération nerveuse, ligamentaire et/ou tendineuse et/ou valve cardiaque artificielle, produits à partir de tissus biologiques et répondant à l'ensemble des problématiques ci-dessus exposées.

Dans ce contexte, la membrane amniotique, obtenue à partir du placenta après un accouchement, est un tissu utilisé depuis plus de cent ans dans le traitement des brûlures et des blessures. En effet, dès 1909, Davies (Davis JS. II. Skin Grafting at the Johns Hopkins Hospital. Ann Surg. 1909 Sep;50(3):542-9. doi: 10.1097/00000658-190909000-00002. PMID: 17862406; PMCID: PMC1407162.) utilisait des membranes fœtales tant sur des brûlures que sur des tissus ulcérés. En 1972, les travaux de Trelford *et al.,* (Trelford JD, Anderson DG, Hanson FW, Mendel V, Sawyer RH. Amnion autografts and allografts as a cover for skin defects in sheep. A preliminary report. J Med. 1972;3(2):81-7. PMID: 4507072.) ont confirmé ce fait. Fénelon *et al.,* (Fénelon M, Catros S, Meyer C, Fricain JC, Obert L, Auber F, Louvrier A, Gindraux F. Applications of Human Amniotic Membrane for Tissue Engineering. Membranes (Basel). 2021 May 25;11(6):387. doi: 10.3390/membranes11060387. PMID: 34070582; PMCID: PMC8227127.) rapportent qu'à partir de 1972, et surtout depuis sa redécouverte en 1995, d'autres auteurs ont confirmé toutes les applications cliniques présentées précédemment, et ont également signalé de nouvelles indications telles que le tractus génito-urinaire, l'estomac, le larynx, la cavité orale, la tête et le cou, que ce soit dans des essais cliniques ou des rapports de cas.

La demande EP4209200A1 divulgue un procédé de fabrication d'un dispositif utilisable comme lentille à partir d'une membrane amniotique placentaire comprenant une structure annulaire réticulée uniquement à la périphérie de celle-ci de manière à disposer d'une structure externe rigide moins fragile que la seule membrane amniotique placentaire et plus simple à mettre en place sur le patient, tout en ayant une excellente biocompatibilité. La partie centrale constituée de membrane amniotique placentaire n'est pas réticulée.

En effet, en raison de son épaisseur comprise entre 30 à 50 µm, la membrane amniotique placentaire est fragile et trop souple pour être manipulée sans supports. Cette finesse limite par ailleurs la création d'autres dispositifs différents par le même procédé. A l'inverse, la présente invention est relative à un procédé mettant en œuvre un matériau de départ différent, la paroi de cordon ombilical dont l'épaisseur est comprise entre 0,5 mm et 2mm et un procédé comprenant une étape traitement avec une base forte pendant au moins une heure et une étape de de réticulation sur l'ensemble de la structure permettant d'obtenir un produit final plus épais et aux propriétés mécaniques supérieures permettant la réalisation des différents dispositifs selon l'invention.

La demande US2015335771 divulgue une méthode de stérilisation d'un produit de tissu de soutien fœtal. Ce tissu de soutien fœtal peut être sélectionné parmi une première liste comprenant de la membrane amniotique placentaire, de la membrane amniotique du cordon ombilical, du chorion, de l'amnios-chorion, du placenta, du cordon ombilical ou de toute combinaison de ceux-ci.

Elle mentionne également dans une seconde liste de nombreuses applications potentielles de ces tissus. Aucune de ces applications n'est spécifiquement identifiée comme pouvant être réalisée avec de la paroi de cordon ombilical.

En outre, cette demande ne divulgue pas non plus les étapes essentielles de la présente invention, et en particulier ni les étapes de traitement avec une base forte, ni de réticulation.

A l'inverse, cette demande ne divulgue que des étapes de stérilisation du produit de tissu de soutien fœtal. Le produit obtenu ne présente donc pas les mêmes propriétés que celui selon l'invention.

La demande WO2008042441 divulgue un procédé d'obtention de biomatériaux issus de membrane de cordon ombilical et leur utilisation en tant que complément aux chirurgies oculaires. Le procédé divulgué dans cette demande ne comprend pas d'étape de traitement prolongé avec une base forte. Le procédé divulgué a pour objectif de préserver les propriétés biomécaniques et structurelles des composants de ce dernier, en particulier du collagène. Les matériaux obtenus ne présentent donc pas la rigidité requise pour les différentes applications visées par la présente invention.

La demande WO2008060377 divulgue l'utilisation d'une composition étant préférentiellement sous la forme d'une solution, d'une suspension, d'un gel ou d'une pâte. A l'inverse, le procédé selon la présente invention permet de conserver une l'intégrité structurelle de la membrane amniotique assurant ainsi une meilleure tenue dans le temps de la forme.

La demande US2019192734 divulgue un procédé de déshydratation de tissus placentaires pour la production de tissus placentaires utilisables pour la cicatrisation des tissus. Elle décrit en particulier un procédé de déshydratation de cordon ombilical ne comprenant pas les étapes selon la présente invention pour la formation d'une structure décrite comme fine et pliable à l'inverse de l'épaisseur et la rigidité recherchée

La demande KR20090006256 divulgue un procédé de traitement d'une matrice de collagène de manière à la rendre transparente pour son utilisation en tant que greffe de cornée. Cette matrice est obtenue à partir de tissu cutané ou de tissu ombilical. Cette demande ne divulgue cependant pas l'utilisation de la membrane amniotique de cordon ombilical. Son procédé comprend une première étape de traitement enzymatique du tissu cible avec une collagénase et/ou une protéase de manière à dénaturer puis reconstituer une nouvelle matrice de collagène par la suite réticulée.

Elle propose donc un procédé dont la finalité est l'inverse de celle selon la présente invention. En effet, dans le procédé selon l'invention, la structure native des protéines de la matrice extracellulaire et en particulier du collagène de la paroi de cordon ombilical n'est pas altérée et est à l'inverse conservée par réticulation. Le produit obtenu présente une organisation structurelle native des fibres de collagène conférant une épaisseur suffisante ainsi que des qualités de résistance et compatible avec les différentes applications visées.

La demande US2017049928A1 divulgue un produit de transplantation dérivé du cordon ombilical humain destiné à être utilisé comme barrière pour les tissus mous, comme revêtement de plaie ou comme autre accessoire de cicatrisation interne ou externe. Le procédé de fabrication de ce produit de transplantation consiste en un lavage et un séchage du dérivé de cordon ombilical avant implantation. Le produit obtenu selon cette demande est donc très souple et nécessite une étape de suture pour être maintenu autour des zones et organes traités.

A l'inverse le produit selon la présente invention, en particulier lorsqu'il est utilisé en tant que guide de régénération nerveuse, ligamentaire et/ou tendineuse, présente une rigidité suffisante pour conserver une forme tubulaire compatible avec cette utilisation et ne nécessite donc pas de suture pour être maintenu sur les zones et organes traités.

Il existe donc un besoin de disposer de structures ayant une taille, notamment une épaisseur, et des propriétés mécaniques notamment de rigidité, compatibles avec les applications visées.

De manière particulièrement surprenante, la demanderesse est parvenue à mettre au point un dispositif constitué de paroi de cordon dont l'épaisseur est comprise entre 0,5 mm et 2 mm. Les étapes du procédé de fabrication confèrent audit dispositif une résistance suffisante pour être manipulable et utilisable, sans risque de détérioration, sans structure de support.

Ledit procédé selon la présente invention permet donc la fabrication de dispositifs plus épais notamment que les dispositifs obtenus à partir de membrane amniotique placentaire et par ailleurs utilisables pour la fabrication des divers dispositifs implantables suscités.

De manière particulièrement surprenante, la demanderesse a en particulier mis au point une étape de traitement prolongé par une base forte permettant d'améliorer les propriétés de résistance du biomatériau, en particulier à la suite de l'étape de réticulation.

Le procédé selon la présente invention présente l'avantage de conserver l'intégrité de la surface de la paroi de cordon ombilical. Il n'entraine en particulier pas de rupture aléatoire de la continuité de la paroi de cordon ombilical ni de décellularisation de ladite paroi, lui conférant des propriétés mécaniques supérieures à celles connues de l'art antérieur.

Il permet, en outre, de conserver l'organisation native des molécules de la matrice extracellulaire de la paroi de cordon ombilical, en particulier du collagène.

La figure 1 est une photographie d'un dispositif implantable consistant en une lentille obtenue par le procédé selon l'invention.

La figure 2 est une photographie d'un dispositif implantable consistant en un guide de régénération nerveuse, ligamentaire et/ou tendineuse obtenu par le procédé selon l'invention.

La figure 3 est une photographie d'un dispositif implantable consistant en une valve cardiaque artificielle obtenue par le procédé selon l'invention.

Les figure 4 et figure 5 sont des représentations schématiques de l'étape w') de mise en forme du dispositif implantable constitué de paroi de cordon ombilical, ledit dispositif implantable étant une valve cardiaque artificielle.

La présente invention concerne un procédé de fabrication d'un dispositif implantable caractérisé en ce qu'il comprend les étapes de :
a) On dispose d'une paroi de cordon ombilical,
b) On traite la paroi de cordon ombilical avec une base forte pendant une durée d'au moins une heure,
c) On procède à au moins une étape de réticulation,
d) On obtient un dispositif implantable consistant en une paroi de cordon ombilical réticulée,
ledit procédé ne comprenant pas d'étape de traitement de la paroi de cordon ombilical par des protéases et/ou des collagénases.

Dans un mode de réalisation, le dispositif implantable est choisi dans le groupe comprenant une lentille biologique, un implant intra-cornéen consistant en un anneau ou un segment anneau, un guide de régénération nerveuse, ligamentaire et/ou tendineuse et/ou une valve cardiaque artificielle.

Dans un mode de réalisation, le procédé selon la présente invention est caractérisé en ce qu'il permet de conserver l'intégrité de la surface de la paroi de cordon ombilical.

Au sens de la présente invention, on entend par « conserver l'intégrité de la surface » l'absence de rupture aléatoire de la continuité de la surface, en particulier par broyage, homogénéisation, fragmentation et/ou pulvérisation de la paroi de cordon ombilical, étant entendu que la paroi pourra être notamment découpée pour la mettre aux dimensions et à la forme du dispositif implantable.

Dans un mode de réalisation, le procédé selon la présente invention ne comprend pas d'étape entrainant la décellularisation de la paroi amniotique de cordon ombilical.

Dans un mode de réalisation, le procédé comprend au moins une étape de découpe de la paroi pour la mettre aux dimensions et à la forme du dispositif implantable.

Au sens de la présente invention, on entend par « paroi de cordon ombilical » un cordon ombilical exempt de vaisseaux, comprenant au moins la membrane amniotique de cordon ombilical.

Dans un mode de réalisation, la paroi de cordon ombilical dont on dispose à l'étape a) est essentiellement exempte de gelée de Wharton.

Dans un mode de réalisation, la paroi de cordon ombilical dont on dispose à l'étape a) est exempte de gelée de Wharton.

Dans un mode de réalisation, la paroi de cordon ombilical dont on dispose à l'étape a) comprend de la gelée de Wharton.

Dans un mode de réalisation, la paroi de cordon ombilical dont on dispose à l'étape a) comprend une épaisseur de gelée de Wharton comprise entre 0,3 et 3 millimètres.

Dans un mode de réalisation, la paroi de cordon ombilical dont on dispose à l'étape a) comprend une épaisseur de gelée de Wharton comprise entre 0,3 et 1 millimètre.

Dans un mode de réalisation, la paroi de cordon ombilical dont on dispose à l'étape a) comprend une épaisseur de gelée de Wharton comprise entre 1 et 3 millimètres.

Dans un mode de réalisation, le procédé comprend une étape préalable de préparation d'une paroi de cordon ombilical par obtention d'un cordon ombilical et de retrait mécanique des vaisseaux.

Dans un mode de réalisation, la paroi de cordon ombilical dont on dispose à l'étape a) est d'origine animale ou humaine.

Dans un mode de réalisation, la paroi de cordon ombilical dont on dispose à l'étape a) est d'origine humaine.

Dans un mode de réalisation, la paroi de cordon ombilical dont on dispose à l'étape a) est obtenue selon le protocole tel que décrit dans la demande WO2017140914A1.

La paroi de cordon ombilical selon la présente invention est obtenue après l'accouchement d'une donneuse proprement informée et consentante en accord avec exigences des directives Européennes et la Loi Bioéthique. Cet accouchement peut s'être déroulé par césarienne ou par voie basse.

Par exigence sanitaire relative aux dons de tissus et de cellules d'origine humaine, une qualification socio-clinique du donneur et biologique en amont du donneur est systématique. Cette qualification implique une recherche de virus HIV, hépatites B, C, HTLV et de la bactérie tréponème pâle responsable de la syphilis.

Dans un mode de réalisation, la paroi de cordon ombilical dont on dispose à l'étape a) est préalablement congelée.

Dans un mode de réalisation, la paroi de cordon ombilical dont on dispose à l'étape a) est préalablement placée dans une boîte stérile, congelée et transportée à une température de -20°C, puis stocké à -80°C.

Dans un mode de réalisation, la paroi de cordon ombilical dont on dispose à l'étape a) est transportée à +4 °C.

Dans un mode de réalisation, la paroi de cordon ombilical dont on dispose à l'étape a) n'a pas été congelée initialement, la découpe et la séparation de la paroi de cordon ombilical sont réalisées après le transport à +4°C.

Dans un mode de réalisation, la paroi de cordon ombilical dont on dispose à l'étape a) est conservée à -80°C.

Dans un mode de réalisation, la paroi de cordon ombilical dont on dispose à l'étape a) est viro-inactivée.

Dans un mode de réalisation, le procédé selon l'invention est caractérisé en ce qu'il comprend en outre une étape a') de viro-inactivation de la paroi de cordon ombilical suite à l'étape a).

Dans un mode de réalisation, l'étape a') de viro inactivation comprend une étape i) de viro-inactivation comprenant l'application sur la paroi de cordon ombilical d'un lavage et/ou du séjour de cette dernière dans un bain, composé d'un premier agent viro-inactivant.

Dans un mode de réalisation, le premier agent viro-inactivant est l'éthanol.

Dans un mode de réalisation, le premier agent viro-inactivant est l'éthanol à une teneur en alcool de 50% à 80% (v/v) (50%≤ teneur en alcool ≤80%).

Dans un mode de réalisation, le premier agent viro-inactivant est l'éthanol à une teneur en alcool de 70% (v/v).

Dans un mode de réalisation, l'étape i) de viro-inactivation est effectuée pendant une durée comprise dans un intervalle allant de 30 minutes à 120 minutes (30 min≤ durée ≤120 min).

Dans un mode de réalisation, l'étape i) de viro-inactivation est effectuée pendant une durée comprise dans un intervalle allant de 45 minutes à 100 minutes (45 min≤ durée ≤100 min).

Dans un mode de réalisation, l'étape i) de viro-inactivation est effectuée pendant une durée d'environ 60 minutes.

Dans un mode de réalisation, l'étape i) de viro-inactivation est effectuée en traitant la paroi de cordon ombilical avec de l'éthanol à une teneur en alcool de 70% (v/v) pendant une durée d'environ 60 minutes.

Dans un mode de réalisation, l'étape i) est suivi d'une étape i') de lavage de la paroi de cordon ombilical par de l'eau purifiée ou un séjour dans un bain d'eau purifiée.

Dans un mode de réalisation, l'étape a') de viro inactivation comprend une seconde étape ii) de viro inactivation consécutive à l'étape i) ou i') comprenant l'application à la paroi de cordon ombilical d'un lavage et/ou le séjour de cette dernière dans un bain, composé d'un deuxième agent de viro-inactivation.

Dans un mode de réalisation, le deuxième agent de viro inactivation est le peroxyde d'hydrogène.

Dans un mode de réalisation, le deuxième agent de viro-inactivation est le peroxyde d'hydrogène sous une forme choisie parmi une solution aqueuse et un gaz.

Dans un mode de réalisation, le deuxième agent de viro-inactivation est le peroxyde d'hydrogène sous forme de solution aqueuse dans une concentration comprise dans un intervalle allant de 1% à 30% (w/v) (1% ≤ concentration w/v ≤ 30%).

Dans un mode de réalisation, le deuxième agent de viro-inactivation est le peroxyde d'hydrogène sous forme de solution aqueuse dans une concentration comprise dans un intervalle allant de 3% à 10% (w/v) (3% ≤ concentration w/v ≤ 10%).

Dans un mode de réalisation, l'étape ii) de viro-inactivation est effectuée pendant une durée comprise dans un intervalle allant de 30 minutes à 120 minutes (30 mn≤ durée ≤120 mn).

Dans un mode de réalisation, l'étape ii) de viro-inactivation est effectuée pendant une durée comprise dans un intervalle allant de 45 à 100 minutes (45 mn≤ durée ≤100 mn).

Dans un mode de réalisation, l'étape ii) de viro-inactivation est effectuée pendant une durée d'environ 60 minutes.

Dans un mode de réalisation, le procédé est caractérisé en ce que la deuxième étape de viro-inactivation ii) de la paroi de cordon ombilical comprend deux sous-étapes de traitement par le deuxième agent de viro-inactivation choisi dans la famille des peroxydes.

Au sens de la présente invention, on entend par « peroxydes » tout composé chimique contenant un groupe fonctionnel de formule générale R-O-O-R' (deux atomes d'oxygène voisins liés entre eux) où R et R' sont des atomes d'hydrogène ou des radicaux alkyls quelconques.

Selon un mode de réalisation, la deuxième étape de viro-inactivation ii) de la paroi de cordon ombilical comprend deux sous-étapes de traitement par le peroxyde d'hydrogène :
une première sous-étape de traitement ii') s'effectuant avec une solution de peroxyde d'hydrogène à une concentration supérieure à 10% w/v pendant une durée d'au moins de 20 minutes ; et
une deuxième sous-étape de traitement ii") s'effectuant avec une solution de peroxyde d'hydrogène à une concentration inférieure à 5 % w/v pendant une durée supérieure à 50 minutes.

Selon un autre mode de réalisation, la deuxième étape de viro-inactivation ii) la paroi de cordon ombilical comprend deux sous-étapes de traitement par le peroxyde d'hydrogène :
une première sous-étape de traitement ii') s'effectuant avec une solution de peroxyde d'hydrogène à une concentration de 30% w/v pendant une durée d'environ 15 minutes ; et
une deuxième sous-étape de traitement ii')' s'effectuant avec une solution de peroxyde d'hydrogène à une concentration de 3% w/v pendant une durée d'environ 60 minutes.

Dans un mode de réalisation, l'étape a') de viro inactivation comprend une troisième étape iii) consécutive à l'étape ii) comprenant au moins une étape de neutralisation, par application à la paroi de cordon ombilical comprend d'un lavage et/ ou d'un séjour de cette dernière dans un bain, composé d'au moins un tampon basique.

Dans un mode de réalisation, le tampon basique est ajouté en une quantité suffisante pour neutraliser le pH dans troisième étape iii).

Dans un mode de réalisation, l'étape a') de viro inactivation comprend une dernière étape iv) comprenant l'application à la paroi de cordon ombilical d'un lavage et/ou d'un séjour dans un bain, composé d'au moins une solution tampon assurant un rééquilibrage physiologique.

Dans un mode de réalisation, la dernière étape iv) comprend deux bains effectués en solution de tampon phosphate salin (PBS).

Dans un mode de réalisation, les étapes de décongélation, viro-inactivation, lavage, ajustement de pH et mise en tampon s'effectuent à température ambiante.

A l'étape b) du procédé, la paroi est traitée par une base forte. Au sens de la présente invention, on entend par « base forte » une solution aqueuse d'un sel alcalin dont le pKa est supérieur à 14.

Dans un mode de réalisation, l'étape b) est caractérisée en ce que la base forte est une solution aqueuse d'un sel alcalin choisi dans le groupe comprenant l'hydroxyde de sodium, l'hydroxyde de potassium, l'hydroxyde de calcium, l'hydroxyde de magnésium, l'hydroxyde de lithium, l'hydroxyde d'ammonium, l'hydroxyde de tétraméthylammonium, l'hydroxyde de triméthylbenzylammonium, l'hydroxyde de baryum et/ou l'hydroxyde de strontium.

Dans un mode de réalisation, l'étape b) est caractérisée en ce que le sel alcalin est l'hydroxyde de sodium.

Dans un mode de réalisation, l'étape b) est caractérisée en ce que la concentration en sel alcalin est comprise dans un intervalle allant de 0,1M à 5M.

Dans un mode de réalisation, l'étape b) est caractérisée en ce que la concentration en sel alcalin est comprise dans un intervalle allant de 0,5M à 3M.

Dans un mode de réalisation, l'étape b) est caractérisée en ce que la concentration en sel alcalin est comprise dans un intervalle allant de 1M à 2M.

Dans un mode de réalisation, l'étape b) est caractérisée en ce que la concentration en sel alcalin est d'environ 1M.

Dans un mode de réalisation, l'étape b) est caractérisée en ce qu'elle est réalisée pendant une durée d'une heure à 48 heures.

Dans un mode de réalisation, l'étape b) est caractérisée en ce qu'elle est réalisée pendant une durée d'au moins 2 heures.

Dans un mode de réalisation, l'étape b) est caractérisée en ce qu'elle est réalisée pendant une durée comprise dans un intervalle allant de 8h à 48h à une température d'environ 4°C.

Dans un mode de réalisation, l'étape b) est caractérisée en ce qu'elle est réalisée pendant une durée comprise dans un intervalle allant de 12h à 36h à une température d'environ 4°C.

Dans un mode de réalisation, l'étape b) est caractérisée en ce qu'elle est réalisée pendant une durée comprise dans un intervalle allant de 16h à 24h à une température d'environ 4°C.

Dans un mode de réalisation, l'étape b) est caractérisée en ce qu'elle est réalisée pendant une durée d'environ 16h, 17h, 18h, 19h, 20h, 21h ou 22h à une température d'environ 4°C.

Dans un mode de réalisation, l'étape b) est caractérisée en ce qu'elle est réalisée pendant une durée comprise dans un intervalle allant de 1h à 4h à une température comprise entre 20°C et 27°C.

Dans un mode de réalisation, l'étape b) est caractérisée en ce qu'elle est réalisée pendant une durée comprise dans un intervalle allant de 1h à 3h à une température comprise entre 20°C et 27°C.

Dans un mode de réalisation, l'étape b) est caractérisée en ce qu'elle est réalisée sous agitation faible.

Dans un mode de réalisation, le procédé selon l'invention est caractérisé en ce qu'il comprend en outre une étape b') de neutralisation du pH avant l'étape c) de réticulation.

Dans un mode de réalisation, le procédé selon l'invention est caractérisé en ce que l'étape b') de neutralisation est réalisée par au moins un lavage à l'eau.

Dans un mode de réalisation, l'étape c) de réticulation est caractérisée en ce qu'elle est réalisée selon le protocole tel que décrit dans la demande EP4209200A1.

Dans un mode de réalisation, l'étape c) de réticulation est caractérisée en ce qu'elle est réalisée par glycation non enzymatique, irradiation par des rayons UV avec ou sans un agent photosensibilisateur et/ou par réaction aldéhydique.

Dans un mode de réalisation, l'étape c) de réticulation est caractérisée en ce qu'elle est réalisée par irradiation par des rayons UV avec ou sans un agent photosensibilisateur.

Dans un mode de réalisation, l'étape c) de réticulation est caractérisée en ce qu'elle est réalisée par irradiation par des rayons UV-A et/ou UV-C avec ou sans un agent photosensibilisateur.

Dans un mode de réalisation, l'étape c) de réticulation est caractérisée en ce qu'elle est réalisée par irradiation par des rayons UV avec un agent photosensibilisateur.

Dans un mode de réalisation de l'un quelconque des procédés selon l'invention, l'étape c) de réticulation est réalisée par irradiation par des rayons UV en présence de riboflavine ou d'un de ses sels.

Dans un mode de réalisation, l'étape c) de réticulation est réalisée par irradiation par des rayons UV-A en présence de sel de sodium phosphaté de riboflavine.

La riboflavine et le sel de sodium phosphaté de riboflavine sont des molécules photosensibilisantes et photopolymérisantes qui ont une capacité de diffusion faible. La riboflavine est un constituant essentiel des cellules vivantes et est non cytotoxique, son utilisation sera donc sans aucune conséquence sur la toxicité des dispositifs ainsi fabriqués.

Dans un mode de réalisation, l'étape c) de réticulation est caractérisée en ce que la concentration en riboflavine ou en sel de riboflavine est comprise dans un intervalle allant de 0,01 g/L à 2 g/L.

Dans un mode de réalisation, l'étape c) de réticulation est caractérisée en ce que la concentration en riboflavine ou en sel de riboflavine est comprise dans un intervalle allant de 0,1 g/L à 0,5 g/L.

Dans un mode de réalisation, l'étape c) de réticulation est caractérisée en ce que la concentration en riboflavine ou en sel de riboflavine d'environ 0,1 g/L.

Dans un mode de réalisation, l'étape c) de réticulation est caractérisée par l'application d'un rayonnement UV de compris entre 10 et 20 joules pendant 15 à 60 minutes.

Dans un mode de réalisation, l'étape c) de réticulation est caractérisée par l'application d'un rayonnement UV-A de compris entre 10 et 20 joules pendant 2 fois 15 minutes.

Dans un mode de réalisation, l'étape c) de réticulation est caractérisée par l'application d'un rayonnement UV de compris entre 12 et 16 joules pendant 20 à 40 minutes.

Dans un mode de réalisation, l'étape c) de réticulation est caractérisée par l'application d'un rayonnement UV de 14,4 joules pendant 30 minutes

Dans un mode de réalisation, le procédé selon l'invention est caractérisé en ce qu'il comprend en outre une étape c') de rinçage, consécutive à l'étape c).

Cette étape permet d'assurer l'élimination de tout l'agent réticulant.

Dans un mode de réalisation, l'étape c') de rinçage est réalisée avec de l'eau.

Dans un mode de réalisation, l'étape c') de rinçage est réalisée avec un mélange d'eau et de macromolécules choisies parmi le dextrane, la carboxyméthyl cellulose et/ou le polyvinylpyrrolidone (PVP).

Dans un mode de réalisation, l'étape c') de rinçage est réalisée avec un mélange d'eau et de dextrane.

Dans un mode de réalisation, l'étape c') de rinçage est réalisée avec un mélange d'eau et dextrane à une concentration comprise dans un intervalle allant de 0,1% à 10%.

Dans un mode de réalisation, l'étape c') de rinçage est réalisée avec un mélange d'eau et dextrane à une concentration comprise dans un intervalle allant de 1% à 8%.

Dans un mode de réalisation, l'étape c') de rinçage est réalisée avec un mélange d'eau et dextrane à une concentration comprise dans un intervalle allant de 3% à 7%.

Dans un mode de réalisation, l'étape c') de rinçage est réalisée avec un mélange d'eau et de carboxyméthyl cellulose.

Dans un mode de réalisation, l'étape c') de rinçage est réalisée avec un mélange d'eau et de carboxyméthyl cellulose à une concentration comprise dans un intervalle allant de 0,1% à 10%.

Dans un mode de réalisation, l'étape c') de rinçage est réalisée avec un mélange d'eau et de carboxyméthyl cellulose à une concentration comprise dans un intervalle allant de 1% à 5%.

Dans un mode de réalisation, le procédé selon l'invention est caractérisé en ce qu'il comprend en outre une étape y) de séchage à la suite de l'étape c), ou alternativement à la suite de l'étape c') lorsque cette étape est mise en œuvre.

Dans un mode de réalisation, l'étape y) de séchage est caractérisée en ce qu'elle comprend au moins une étape de dessication et/ou de lyophilisation.

Dans un mode de réalisation, l'étape y) de séchage est caractérisée en ce qu'elle comprend au moins une étape de dessication.

Au sens de la présente invention, on entend par « dessication » l'élimination de l'eau contenue dans une substance par vaporisation, à l'aide de la chaleur, du vide et/ou d'une matière hygroscopique.

Dans un mode de réalisation, l'étape y) de séchage par dessication comprend l'application de chaleur, de vide et/ou d'une matière hygroscopique.

Dans un mode de réalisation, l'étape y) de séchage par dessication comprend l'application d'une part de la chaleur et d'autre part de vide et/ou d'une matière hygroscopique.

Dans un mode de réalisation, l'étape y) de séchage par dessication comprend l'application de chaleur et de vide.

Dans un mode de réalisation, l'étape y) de séchage par dessication ne comprend pas de congélation et/ou de surgélation de la paroi de cordon ombilical.

Dans un mode de réalisation, dans l'étape y) de séchage par dessication, la température minimale est choisie de manière à éviter la formation de cristaux d'eau en fonction de la pression appliquée.

Dans un mode de réalisation, l'étape y) de séchage par dessication est réalisée par montée progressive de la température à partir d'au moins 0°C (0°C ≤ température).

Dans un mode de réalisation, l'étape y) de séchage par dessication est réalisée par montée progressive de la température à partir d'au moins 2°C (2°C ≤ température).

Dans un mode de réalisation, l'étape y) de séchage par dessication est réalisée par montée progressive de la température à partir d'au moins 4°C (4°C ≤ température).

Dans un mode de réalisation, l'étape y) de séchage par dessication est réalisée par montée progressive de la température jusqu'à 35°C.

Dans un mode de réalisation, l'étape y) de séchage par dessication est réalisée par montée progressive de la température d'au moins 0°C jusqu'à 35°C.

Dans un mode de réalisation, l'étape y) de séchage par dessication est réalisée par montée progressive de la température d'au moins 0°C à 35°C (0°C ≤ température ≤ 35°C).

Dans un mode de réalisation, l'étape y) de séchage par dessication est réalisée par montée progressive de la température dans un intervalle allant de 4°C à 35°C (4°C ≤ température ≤ 35°C).

Dans un mode de réalisation, l'étape y) de séchage par dessication est réalisée par montée progressive de la température par paliers de 1 à 10°C (1°C ≤ température ≤ 10°C).

Dans un mode de réalisation, l'étape y) de séchage par dessication est réalisée par montée progressive de la température par palier de 1 à 10°C.

Dans un mode de réalisation, l'étape y) de séchage par dessication est réalisée par montée progressive de la température par palier de 2 à 8°C.

Dans un mode de réalisation, l'étape y) de séchage par dessication est réalisée par montée progressive de la température par palier de 3 à 7°C.

Dans un mode de réalisation, l'étape y) de séchage par dessication est réalisée par montée progressive de la température par palier de 5°C.

Dans un mode de réalisation, l'étape y) de séchage par dessication est réalisée par montée progressive de la température de 5°C.

Dans un mode de réalisation, l'étape y) de séchage par dessication est réalisée pendant une durée comprise dans un intervalle allant de 1 heure à 12 heures.

Dans un mode de réalisation, l'étape y) de séchage par dessication est réalisée pendant une durée comprise dans un intervalle allant de 2 heures à 10 heures.

Dans un mode de réalisation, l'étape y) de séchage par dessication est réalisée pendant une durée comprise dans un intervalle allant de 3 heures à 7 heures.

Dans un mode de réalisation, l'étape y) de séchage par dessication est réalisée pendant une durée comprise dans un intervalle allant de 4 heures à 6 heures.

Dans un mode de réalisation, l'étape y) de séchage par dessication est réalisée sous des pressions comprises dans un intervalle allant de 50 à 900 micro-bars (50 micro-bars ≤ pressions ≤ 900 micro-bars).

Dans un mode de réalisation, l'étape y) de séchage par dessication est réalisée sous des pressions comprises dans un intervalle allant de 100 à 800 micro-bars (100 micro-bars ≤ pressions ≤ 800 micro-bars).

Dans un mode de réalisation, l'étape y) de séchage par dessication est réalisée sous des pressions comprises dans un intervalle allant de 400 à 600 micro-bars (400 micro-bars ≤ pressions ≤ 600 micro-bars).

Dans un mode de réalisation, l'étape y) de séchage par dessication est réalisée sous des pressions d'environ 600 micro-bars.

Dans un mode de réalisation, l'étape y) de séchage par dessication est réalisée sous des pressions d'environ 400 micro-bars.

Dans un mode de réalisation, l'étape y) de séchage par dessication est réalisée par montée progressive de la température jusqu'à 35°C et sous des pressions comprises dans un intervalle allant de 50 à 900 micro-bars (50 micro-bars ≤ pressions ≤ 900 micro-bars).

Dans un mode de réalisation, l'étape y) de séchage par dessication est réalisée par montée progressive de la température d'au moins 0°C jusqu'à 50°C et sous des pressions comprises dans un intervalle allant de 50 à 900 micro-bars (50 micro-bars ≤ pressions ≤ 900 micro-bars).

Dans un mode de réalisation, l'étape y) de séchage par dessication est réalisée par montée progressive de la température d'au moins 2°C jusqu'à 45°C et sous des pressions comprises dans un intervalle allant de 50 à 900 micro-bars (50 micro-bars ≤ pressions ≤ 900 micro-bars).

Dans un mode de réalisation, l'étape y) de séchage par dessication est réalisée par montée progressive de la température d'au moins 4°C jusqu'à 40°C et sous des pressions comprises dans un intervalle allant de 50 à 900 micro-bars (50 micro-bars ≤ pressions ≤ 900 micro-bars).

Dans un mode de réalisation, l'étape y) de séchage par dessication est réalisée par montée progressive de la température d'au moins 8°C jusqu'à 35°C et sous des pressions comprises dans un intervalle allant de 50 à 900 micro-bars (50 micro-bars ≤ pressions ≤ 900 micro-bars).

Dans un mode de réalisation, l'étape y) de séchage par dessication est réalisée par montée progressive de la température d'au moins 0°C jusqu'à 35°C et sous des pressions comprises dans un intervalle allant de 50 à 900 micro-bars (50 micro-bars ≤ pressions ≤ 900 micro-bars).

Dans un mode de réalisation, l'étape y) de séchage par dessication est réalisée par montée progressive de la température d'au moins 0°C à 35°C (0°C ≤ température ≤ 35°C) et sous des pressions comprises dans un intervalle allant de 50 à 900 micro-bars (50 micro-bars ≤ pressions ≤ 900 micro-bars).

Dans un mode de réalisation, l'étape y) de séchage par dessication est réalisée par montée progressive de la température dans un intervalle allant de 4°C à 35°C (4°C ≤ température ≤ 35°C) et sous des pressions comprises dans un intervalle allant de 50 à 900 micro-bars (50 micro-bars ≤ pressions ≤ 900 micro-bars).

Dans un mode de réalisation, l'étape y) de séchage par dessication est réalisée par montée progressive de la température jusqu'à 35°C et sous des pressions comprises dans un intervalle allant de 100 à 800 micro-bars (100 micro-bars ≤ pressions ≤ 800 micro-bars).

Dans un mode de réalisation, l'étape y) de séchage par dessication est réalisée par montée progressive de la température d'au moins 0°C jusqu'à 50°C et sous des pressions comprises dans un intervalle allant de 100 à 800 micro-bars (100 micro-bars ≤ pressions ≤ 800 micro-bars).

Dans un mode de réalisation, l'étape y) de séchage par dessication est réalisée par montée progressive de la température d'au moins 2°C jusqu'à 45°C et sous des pressions comprises dans un intervalle allant de 100 à 800 micro-bars (100 micro-bars ≤ pressions ≤ 800 micro-bars).

Dans un mode de réalisation, l'étape y) de séchage par dessication est réalisée par montée progressive de la température d'au moins 4°C jusqu'à 40°C et sous des pressions comprises dans un intervalle allant de 100 à 800 micro-bars (100 micro-bars ≤ pressions ≤ 800 micro-bars).

Dans un mode de réalisation, l'étape y) de séchage par dessication est réalisée par montée progressive de la température d'au moins 8°C jusqu'à 35°C et sous des pressions comprises dans un intervalle allant de 100 à 800 micro-bars (100 micro-bars ≤ pressions ≤ 800 micro-bars).

Dans un mode de réalisation, l'étape y) de séchage par dessication est réalisée par montée progressive de la température d'au moins 0°C jusqu'à 35°C et sous des pressions comprises dans un intervalle allant de 100 à 800 micro-bars (100 micro-bars ≤ pressions ≤ 800 micro-bars).

Dans un mode de réalisation, l'étape y) de séchage par dessication est réalisée par montée progressive de la température d'au moins 0°C à 35°C (0°C ≤ température ≤ 35°C) et sous des pressions comprises dans un intervalle allant de 100 à 800 micro-bars (100 micro-bars ≤ pressions ≤ 800 micro-bars).

Dans un mode de réalisation, l'étape y) de séchage par dessication est réalisée par montée progressive de la température dans un intervalle allant de 4°C à 35°C (4°C ≤ température ≤ 35°C) et sous des pressions comprises dans un intervalle allant de 100 à 800 micro-bars (100 micro-bars ≤ pressions ≤ 800 micro-bars).

Dans un mode de réalisation, l'étape y) de séchage par dessication est réalisée par montée progressive de la température jusqu'à 35°C et sous des pressions comprises dans un intervalle allant de 400 à 600 micro-bars (400 micro-bars ≤ pressions ≤ 600 micro-bars).

Dans un mode de réalisation, l'étape y) de séchage par dessication est réalisée par montée progressive de la température d'au moins 0°C jusqu'à 50°C et sous des pressions comprises dans un intervalle allant de 400 à 600 micro-bars (400 micro-bars ≤ pressions ≤ 600 micro-bars).

Dans un mode de réalisation, l'étape y) de séchage par dessication est réalisée par montée progressive de la température d'au moins 2°C jusqu'à 45°C et sous des pressions comprises dans un intervalle allant de 400 à 600 micro-bars (400 micro-bars ≤ pressions ≤ 600 micro-bars).

Dans un mode de réalisation, l'étape y) de séchage par dessication est réalisée par montée progressive de la température d'au moins 4°C jusqu'à 40°C et sous des pressions comprises dans un intervalle allant de 400 à 600 micro-bars (400 micro-bars ≤ pressions ≤ 600 micro-bars).

Dans un mode de réalisation, l'étape y) de séchage par dessication est réalisée par montée progressive de la température d'au moins 8°C jusqu'à 35°C et sous des pressions comprises dans un intervalle allant de 400 à 600 micro-bars (400 micro-bars ≤ pressions ≤ 600 micro-bars).

Dans un mode de réalisation, l'étape y) de séchage par dessication est réalisée par montée progressive de la température d'au moins 0°C jusqu'à 35°C et sous des pressions comprises dans un intervalle allant de 400 à 600 micro-bars (400 micro-bars ≤ pressions ≤ 600 micro-bars).

Dans un mode de réalisation, l'étape y) de séchage par dessication est réalisée par montée progressive de la température d'au moins 0°C à 35°C (0°C ≤ température ≤ 35°C) et sous des pressions comprises dans un intervalle allant de 400 à 600 micro-bars (400 micro-bars ≤ pressions ≤ 600 micro-bars).

Dans un mode de réalisation, l'étape y) de séchage par dessication est réalisée par montée progressive de la température dans un intervalle allant de 4°C à 35°C (4°C ≤ température ≤ 35°C) et sous des pressions comprises dans un intervalle allant de 400 à 600 micro-bars (400 micro-bars ≤ pressions ≤ 600 micro-bars).

Dans un mode de réalisation, l'étape y) de séchage est caractérisée en ce qu'elle comprend au moins une étape de lyophilisation.

Dans un mode de réalisation, la lyophilisation est réalisée dans les conditions suivantes :
une congélation en deux étapes :
   - la première étape de congélation s'effectuant à une température d'acclimatation choisie pour ne pas endommager l'intégrité structurelle, fonctionnelle et biologique de la paroi de cordon ombilical,
   - la deuxième étape de congélation s'effectuant à la température finale de congélation qui est inférieure à la température d'acclimatation ;
et, une lyophilisation en deux étapes principales, dites primaire et secondaire :
   - l'étape de lyophilisation primaire s'effectuant par application d'un vide à environ 200 microbars et d'un profil de température ascendant ;
   - l'étape secondaire de lyophilisation s'effectuant par application d'un vide à environ 50 microbars et d'un profil de température descendant.

Dans un mode de réalisation la température d'acclimatation est comprise entre - 5 et - 20°C et la température finale de congélation est comprise entre - 40 et - 60 °C.

Le profil de température ascendant est avantageusement un profil selon lequel la température de lyophilisation est initialement réglée à une température initiale basse puis augmentée vers une température finale de lyophilisation primaire, en une ou plusieurs étapes de température ascendantes intermédiaires. Le profil de température descendant est avantageusement un profil selon lequel la température de lyophilisation est initialement réglée à une température supérieure à la température finale de l'étape de lyophilisation primaire, puis qui est ensuite descendue vers une température finale de lyophilisation secondaire supérieure à la température initiale de l'étape de lyophilisation primaire.

Au sens de la présente invention, on entend par « lyophilisation » une technique visant à dessécher par sublimation un produit préalablement surgelé. Plus précisément, le liquide à ôter du produit est dans un premier temps transformé en solide (glace) par congélation ; puis par une dessiccation primaire, sous vide, le solide est sublimé ; enfin par une dessiccation secondaire, les molécules d'eau à la surface du produit sont extraites par désorption.

Dans un mode de réalisation, le procédé selon l'invention est caractérisé en ce qu'il comprend en outre une étape z) de stérilisation après l'étape c), ou lorsque ces étapes sont mises en œuvre, après l'étape c') ou y).

La stérilisation pourra être réalisée par toute méthode classiquement connue de l'Homme du métier.

Dans un mode de réalisation, l'étape étape z) de stérilisation est réalisée par irradiation.

Dans un mode de réalisation, l'étape étape z) de stérilisation est réalisée par irradiation par des rayonnement gamma.

Dans un mode de réalisation, l'étape étape z) de stérilisation est réalisée par irradiation par des rayonnements gamma à une dose comprise dans un intervalle allant de 25 à 35 kGrays (25 kGrays ≤ dose ≤ 35 kGrays).

Dans un mode de réalisation, le procédé selon l'invention est caractérisé en ce qu'il est réalisé en condition aseptique.

Dans un mode de réalisation, le procédé selon la présente invention est caractérisé en ce qu'il comprend en outre une étape x) de découpe de la paroi de cordon ombilical obtenue à l'issue de l'une quelconque des étapes du procédé.

Dans un mode de réalisation, l'étape x) de découpe est caractérisée en ce qu'elle est effectuée à l'issue de l'une quelconque des étapes a), b) et/ou c).

Dans un mode de réalisation, l'étape x) de découpe est caractérisée en ce qu'elle est effectuée à l'issue de l'une quelconque des étapes a), a'), b), b'), c), c'), y) et/ou z).

Dans un mode de réalisation, l'étape x) de découpe est caractérisée en ce qu'elle est effectuée au moyen d'un instrument et/ou d'un appareil choisi dans le groupe comprenant : un scalpel, une paire de ciseaux chirurgicaux, un microtome, un bistouri électrique, une pince chirurgicale, un laser chirurgical, une lame de rasoir, une scie oscillante, une perforatrice à biopsie et/ou une pince coupante.

Dans un mode de réalisation, l'étape x) de découpe est caractérisée en ce qu'elle est effectuée au moyen d'un laser.

Dans un mode de réalisation, l'étape x) de découpe est caractérisée en ce qu'elle est effectuée au moyen d'un laser chirurgical ou d'un laser industriel de découpe.

Dans un mode de réalisation, l'étape x) de découpe est caractérisée en ce qu'elle est effectuée au moyen d'un laser choisi dans le groupe comprenant : un laser CO₂, un laser à excimères, un laser à diode, un laser Nd, un laser Er, un laser KTP, un laser à argon, un laser à holmium, un laser femtoseconde et/ou un laser thulium.

Dans un mode de réalisation, l'étape x) de découpe est caractérisée en ce qu'elle est effectuée au moyen d'un laser femtoseconde.

Dans un mode de réalisation, l'étape x) de découpe est caractérisée en ce qu'elle est effectuée au moyen d'un laser industriel de découpe de type laser femtoseconde.

Par laser femtoseconde, on entend une source lumineuse, apte à émettre un faisceau L.A.S.E.R. sous forme d'impulsions ultra-courtes, dont la durée est comprise entre 1 femtoseconde et 100 picosecondes.

Dans un mode de réalisation, l'étape x) de découpe est caractérisée en ce qu'elle est effectuée au moyen d'un laser chirurgical de type laser femtoseconde tel que décrit dans la demande WO2016055539 (A1).

La présente invention concerne également un dispositif implantable obtenu par le procédé selon la présente invention.

Dans un mode de réalisation, le dispositif implantable obtenu par le procédé selon la présente invention présente une épaisseur du feuillet de paroi de cordon ombilical comprise entre 0,3 et 3 millimètres.

Dans un mode de réalisation, le dispositif implantable obtenu par le procédé selon la présente invention présente une épaisseur du feuillet de paroi de cordon ombilical comprise entre 0,3 et 1 millimètre.

Dans un mode de réalisation, le dispositif implantable obtenu par le procédé selon la présente invention présente une épaisseur du feuillet de paroi de cordon ombilical comprise entre 1 et 3 millimètres.

Le dispositif implantable peut être une lentille biologique, également appelée « lentille pansement », utilisée notamment pour des applications médicales, par exemple dans le traitement des surfaces oculaires. Par concision, on utilisera uniquement la terminologie « lentille » ci-après.

Du fait de son épaisseur, la lentille ne nécessite pas de structure de support ni de montage double couche pour assurer sa fonction.

Dans un mode de réalisation du procédé selon l'invention, le dispositif implantable est une lentille biologique.

Dans un mode de réalisation, le procédé fabrication d'un dispositif implantable selon l'invention, ledit dispositif implantable étant une lentille, est caractérisé en ce que l'étape x) de découpe est mise en œuvre pour donner une forme de disque, sensiblement circulaire, sensiblement ovale ou elliptique.

Les formes de disque, sensiblement circulaire, sensiblement ovale ou elliptique présentent un diamètre équivalent. Par « diamètre équivalent », on entend l'une des définitions ci-dessous, dépendant de la forme de la paroi de cordon ombilical.

Pour la forme de disque, le diamètre équivalent est le diamètre du cercle représentant la circonférence du disque.

Pour la forme elliptique, le diamètre équivalent est compris entre le double de la longueur du petit axe, également appelé petit rayon et le double de la longueur du grand axe, également appelé grand rayon, de l'ellipse.

Pour la forme ovale sensiblement ovale, c'est à dire une courbe plane fermée convexe, le diamètre équivalent est inférieur ou égal à la longueur de l'axe de symétrie lorsque la courbe plane fermée convexe possède un unique axe de symétrie ; et le diamètre équivalent est compris entre la longueur de l'axe de symétrie le plus long et la longueur de l'axe de symétrie le plus court, lorsque la courbe plane fermée convexe possède deux axes de symétrie orthogonaux.

On prévoit de préférence que le diamètre équivalent de la découpe est inférieur ou égal au diamètre moyen d'un œil humain adulte.

Dans un mode de réalisation, le diamètre équivalent de la découpe est compris dans un intervalle allant de 0,5 à 3 cm.

Dans un mode de réalisation, le diamètre équivalent de la découpe compris dans un intervalle allant de 1 à 2,5 cm.

Dans un mode de réalisation, le procédé fabrication d'un dispositif implantable selon l'invention, ledit dispositif implantable étant une lentille, est caractérisé en ce que l'étape c) de réticulation est réalisée sur un support en forme de dôme.

Dans un mode de réalisation, le support en forme de dôme présente une courbure similaire à celle de la cornée.

Dans un mode de réalisation, le support en forme de dôme est tel que décrit dans la demande WO2020/245324 (A1).

Dans un mode de réalisation, le procédé fabrication d'un dispositif implantable selon l'invention, ledit dispositif implantable étant une lentille, est caractérisé en ce que l'étape de réticulation est réalisée sur un support en forme de dôme, la paroi de cordon ombilical étant disposée de sorte que l'épithélium de la paroi de cordon ombilical est du côté concave.

L'invention concerne également une lentille constituée de paroi de cordon ombilical ayant subi au moins une étape de traitement avec une base forte pendant une durée d'au moins une heure et au moins une étape de réticulation, ladite paroi de cordon ombilical n'étant pas traitée par des protéases et/ou des collagénases.

Dans le mode de réalisation, la réticulation est telle que précédemment décrite.

Dans le mode de réalisation, le traitement avec une base forte pendant une durée d'au moins une heure est tel que précédemment décrit.

Dans un mode de réalisation, lentille selon l'invention est caractérisée qu'elle est viro inactivée.

Dans un mode de réalisation, lentille selon l'invention est caractérisée en ce qu'elle est stérile.

Dans un mode de réalisation, lentille selon l'invention est caractérisée en ce qu'elle est dessiquée.

Dans un mode de réalisation, lentille selon l'invention est caractérisée en ce qu'elle est obtenue par le procédé selon l'invention.

Un autre objet de la présente invention concerne la lentille selon l'invention pour son utilisation thérapeutique.

Un autre objet de la présente invention concerne la lentille selon l'invention pour son utilisation dans le traitement des lésions oculaires et/ou des sècheresses oculaires.

Dans un mode de réalisation, les lésions oculaires et/ou des sècheresses oculaires sont choisies dans le groupe comprenant les maladies inflammatoires de la conjonctive et/ou de la cornée, les infections de la conjonctive et/ou cornée, les destructions de la surface de la conjonctive et/ou cornée, les ulcères cornéens, le syndrome de Stevens-Johnson, le syndrome de Lyell et/ou les kérato-conjonctivites immunitaires, allergiques, inflammatoire et/ou traumatiques.

Dans un mode de réalisation, la lentille selon l'invention est destinée à être utilisé dans le traitement des lésions oculaires et/ou des sècheresses oculaires.

La présente invention concerne également un procédé de traitement d'une lésion oculaire et/ou une sècheresse oculaire chez un sujet nécessitant un tel traitement, ledit procédé comprenant les étapes suivantes :
a) identifier la lésion oculaire et/ou la sècheresse oculaire dans ledit sujet ;
b) fournir une lentille selon l'invention ;
c) appliquer la lentille selon l'invention selon l'invention sur ladite lésion oculaire et/ou une sècheresse oculaire.

Le dispositif implantable peut également être un guide de régénération nerveuse, ligamentaire et/ou tendineuse.

Le guide de régénération nerveuse, ligamentaire et/ou tendineuse selon la présente invention peut être utilisé en chirurgie en le sectionnant éventuellement à sec, de telle sorte que la longueur du dispositif soit d'au moins environ 1 centimètre supérieure à la longueur de l'intervalle à ponter entre les extrémités proximale et distale du tissu à réparer, qui peut être un nerf du système nerveux périphérique, un tendon ou un ligament. Le tissu à réparer est préparé de telle sorte que la section soit nette.

Le guide de régénération nerveuse, ligamentaire et/ou tendineuse selon l'invention est manipulé à sec. Une extrémité (proximale ou distale) du tissu à réparer est insérée à l'intérieur du matériau d'allogreffe viro-inactivé et lyophilisé et stérile, et celui-ci est glissé sur l'extrémité du tissu.

Les deux extrémités du nerf, extrémité proximale et extrémité distale, sont suturées et/ou fixées.

En cas de perte de substance sévère, le tissu à réparer est introduit dans le dispositif implantable à chaque extrémité et stabilisé par suture, le dispositif implantable jouant le rôle de guide.

Le guide de régénération nerveuse, ligamentaire et/ou tendineuse selon l'invention est glissé au-delà de la suture, en le positionnant de part et d'autre de celle-ci, avec les rebords équidistants de la ligne de suture.

Le guide de régénération nerveuse, ligamentaire et/ou tendineuse selon l'invention est naturellement réhydraté par les tissus organiques environnants et s'assouplit.

Comme précédemment indiqué, le guide de régénération nerveuse, ligamentaire et/ou tendineuse selon l'invention, est manipulable à sec. Alternativement, le dispositif est suffisamment rigide pour être manipulé sous arthroscope après réhydratation

Le guide de régénération nerveuse, ligamentaire et/ou tendineuse selon l'invention peut également être utilisé lors de l'utilisation d'une technique d'enrobage, par exemple lorsque le nerf est lésé mais non sectionné ou lorsque l'accès au site d'implantation est difficile.

Ces avantages techniques conférés par un dispositif implantable sont difficilement obtenus à partir d'un produit peu ou pas déformable tel que par exemple les neurotubes synthétiques ou en collagène, en ce que la structure tubulaire sera trop solide pour permettre le passage d'une aiguille de suture courbe à travers la lumière dudit neurotube.

Dans un mode de réalisation, guide de régénération nerveuse, ligamentaire et/ou tendineuse selon l'invention est destiné à être utilisé par une technique d'enrobage de la structure lésée.

Le guide de régénération nerveuse, ligamentaire et/ou tendineuse selon l'invention présente par ailleurs l'avantage d'être suffisamment translucide pour permettre au chirurgien de pouvoir voir et éventuellement le tissu à réparer au travers du guide lors de l'acte chirurgical et ainsi contrôler et éventuellement le repositionner.

Le guide de régénération nerveuse, ligamentaire et/ou tendineuse est également caractérisé en ce que ladite rigidité du guide permet une certaine flexibilité pour pouvoir glisser le long de la structure à traiter et plier lors de son implantation, ainsi que lors des mouvements moteurs du patient ayant subi l'implantation, tout en étant suffisamment rigide pour diminuer les risques de collapsus *in vivo.*

La demanderesse a mis en évidence, par l'observation de coupes histologiques, qu'un guide de régénération nerveuse, ligamentaire et/ou tendineuse selon l'invention présente une structure poreuse adjointe à la surface interne du guide particulièrement appréciée en régénération tissulaire.

Cette rigidité du guide de régénération nerveuse, ligamentaire et/ou tendineuse selon l'invention permet donc la diminution des risques de collapsus, tout en fournissant au tissu destiné à se régénérer au sein dudit guide un réseau poreux longitudinal propice à son développement biologique.

Il présente en outre l'avantage de disposer d'un diamètre interne plus important que les diamètres internes des dispositifs connus de l'art antérieur.

Dans un mode de réalisation du procédé selon l'invention, le dispositif implantable est un guide de régénération nerveuse, ligamentaire et/ou tendineuse.

Dans un mode de réalisation, le procédé fabrication d'un dispositif implantable, ledit dispositif implantable étant un guide de régénération nerveuse, ligamentaire et/ou tendineuse est caractérisé en ce que l'étape x) de découpe est mise en œuvre de sorte à produire une forme de bande.

Dans un mode de réalisation, le procédé fabrication d'un dispositif implantable, ledit dispositif implantable étant un guide de régénération nerveuse, ligamentaire et/ou tendineuse est caractérisé en ce que l'étape x) de découpe est mise en œuvre de sorte à produire une forme de bande entre 1 et 12 cm de longueur.

Dans un mode de réalisation, le procédé fabrication d'un dispositif implantable, ledit dispositif implantable étant un guide de régénération nerveuse, ligamentaire et/ou tendineuse est caractérisé en ce que l'étape x) de découpe est mise en œuvre de sorte à produire une forme de bande de 1 à 5 cm de largeur.

Dans un mode de réalisation, le procédé fabrication d'un dispositif implantable, ledit dispositif implantable étant un guide de régénération nerveuse, ligamentaire et/ou tendineuse est caractérisé en ce que l'étape x) de découpe est mise en œuvre de sorte à produire une forme de bande entre 1 et 12 cm de longueur et de 1 à 5 cm de largeur.

Dans un mode de réalisation, le procédé fabrication d'un dispositif implantable, ledit dispositif implantable étant un guide de régénération nerveuse, ligamentaire et/ou tendineuse est caractérisé en ce que l'étape de réticulation est précédé d'une étape w) d'enroulement de la paroi de cordon ombilical autour d'un support de forme tubulaire, ou sensiblement tubulaire.

Au sens de la présente invention, on entend par « tubulaire » une forme à section circulaire ou sensiblement circulaire, de conduit, de tube ou de cylindre.

Au sens de la présente invention, on entend par « sensiblement circulaire » une forme légèrement ovoïde ou de cercle légèrement aplati. Lorsque la forme est sensiblement circulaire, elle sera définie par un diamètre équivalent.

Dans un mode de réalisation, le support de forme tubulaire, ou sensiblement tubulaire utilisé lors de l'étape w) est caractérisé par un diamètre de 1 à 30 mm.

Dans un mode de réalisation, le support de forme tubulaire, ou sensiblement tubulaire utilisé lors de l'étape w) est caractérisé par un diamètre de 3 à 25 mm.

Dans un mode de réalisation, le support de forme tubulaire, ou sensiblement tubulaire utilisé lors de l'étape w) est caractérisé par un diamètre de 5 à 20 mm.

Dans un mode de réalisation, le support de forme tubulaire, ou sensiblement tubulaire utilisé lors de l'étape w) est caractérisé par un diamètre de 7 à 12 mm.

Selon un mode de réalisation préféré, le support de forme tubulaire, ou sensiblement tubulaire utilisé lors de l'étape w) est en PETG (polyéthylène téréphtalate glycolysé), ou en PET (polyéthylène téréphtalate), ou en PLGA (poly(lactic-co-glycolic acid)), ou en poly(α-hydroxy esters), notamment d'acide polylactique (PLA) et d'acide polyglycolique (PGA), ou différents polyéthylènes.

La présente invention concerne également un guide de régénération nerveuse, ligamentaire et/ou tendineuse constitué de paroi de cordon ombilical ayant subi au moins une étape de traitement avec une base forte pendant une durée d'au moins une heure et au moins une étape de réticulation, ladite paroi de cordon ombilical n'étant pas traitée par des protéases et/ou des collagénases.

Dans un mode de réalisation, le guide de régénération nerveuse, ligamentaire et/ou tendineuse selon la présente invention est caractérisé en ce qu'il est obtenu par le procédé tel que précédemment décrit.

Dans le mode de réalisation, la réticulation est telle que précédemment décrite.

Dans le mode de réalisation, le traitement avec une base forte pendant une durée d'au moins une heure est tel que précédemment décrit.

Dans un mode de réalisation, le guide de régénération nerveuse, ligamentaire et/ou tendineuse selon la présente invention est caractérisé en ce qu'il est viro-inactivé.

Dans un mode de réalisation, le guide de régénération nerveuse, ligamentaire et/ou tendineuse selon la présente invention est caractérisé en ce qu'il est stérile.

Dans un mode de réalisation, le guide de régénération nerveuse, ligamentaire et/ou tendineuse selon la présente invention est caractérisé en ce qu'il est lyophilisé.

Dans un mode de réalisation, le guide de régénération nerveuse, ligamentaire et/ou tendineuse selon la présente invention est caractérisé en ce qu'il est dessiqué.

Dans un mode de réalisation, le guide de régénération nerveuse, ligamentaire et/ou tendineuse selon la présente invention est caractérisé en ce qu'il présente une forme tubulaire non collabable.

Dans un mode de réalisation, le guide de régénération nerveuse, ligamentaire et/ou tendineuse selon la présente invention est caractérisé en ce que le diamètre de sa lumière est compris entre 1,5 à 25 mm.

Dans un mode de réalisation, le guide de régénération nerveuse, ligamentaire et/ou tendineuse selon la présente invention est caractérisé en ce que le diamètre de sa lumière est compris entre 5 à 20 mm.

Dans un mode de réalisation, le guide de régénération nerveuse, ligamentaire et/ou tendineuse selon la présente invention est caractérisé en ce que le diamètre de sa lumière est compris entre 7 à 12 mm.

Dans un mode de réalisation, le guide de régénération nerveuse, ligamentaire et/ou tendineuse selon la présente invention est caractérisé en ce que le diamètre de sa longueur est comprise entre 1 et 12 centimètres.

Dans un mode de réalisation, le guide de régénération nerveuse, ligamentaire et/ou tendineuse selon l'invention est caractérisé en ce qu'il est destiné à être utilisé comme matériau d'allogreffe choisie dans le groupe constitué par la greffe de guide de régénération nerveuse, la greffe de guide de régénération tendineuse, la greffe de guide de régénération ligamentaire.

Dans un mode de réalisation, le guide de régénération nerveuse, ligamentaire et/ou tendineuse est caractérisé en ce qu'il est destiné à être utilisé en tant que matériau d'allogreffe choisie dans le groupe constitué par la réparation d'un nerf du système nerveux périphérique, la réparation du tendon, la réparation du ligament.

La présente invention concerne également un procédé de traitement d'une lésion nerveuse, ligamentaire et/ou tendineuse chez un sujet nécessitant un tel traitement, ledit procédé comprenant les étapes suivantes :
a) identifier la lésion nerveuse, ligamentaire et/ou tendineuse dans ledit sujet ;
b) fournir un guide de régénération nerveuse, ligamentaire et/ou tendineuse selon l'invention ;
c) appliquer le guide de régénération nerveuse, ligamentaire et/ou tendineuse selon l'invention sur ladite lésion.

Dans un mode de réalisation, ledit procédé de traitement d'une lésion nerveuse, ligamentaire et/ou tendineuse selon l'invention est caractérisé en ce que l'étape c) d'application est réalisée par enrobage de la lésion.

Dans un mode de réalisation, ledit procédé de traitement d'une lésion nerveuse, ligamentaire et/ou tendineuse selon l'invention comprend, en outre après l'étape c), une étape d) fixer le guide de régénération nerveuse, ligamentaire et/ou tendineuse selon l'invention autours de la lésion nerveuse, ligamentaire et/ou tendineuse

Dans un mode de réalisation, la lésion nerveuse est choisie dans le groupe comprenant les sections nerveuses traumatiques ou d'exérèse, les névrites, les neurotmésis, les axonotmésis et/ou les neurapraxies.

Dans un mode de réalisation, la lésion ligamentaire est choisie dans le groupe comprenant les entorses et/ou les ruptures totales ou partielles ligamentaires.

Dans un mode de réalisation, la lésion tendineuse est choisie dans le groupe comprenant les tendinites, les tendinoses, les ruptures tendineuses, les enthésopathies et/ou les tendinites calcifiantes.

Le dispositif implantable peut être un implant intra-cornéen consistant en un anneau ou un segment anneau.

Les segments d'anneaux cornéens intra-cornéens sont des dispositifs médicaux utilisés en chirurgie ophtalmologique, principalement pour traiter des anomalies de la cornée, comme le kératocône. Ils sont destinés à être insérés dans la cornée humaine pour en modifier la courbure et corriger de réfraction de l'œil. Ils ont un arc façonné et configuré pour entourer une partie de la cornée lors de son implantation.

Dans un mode de réalisation du procédé selon l'invention, le dispositif implantable est un implant intra-cornéen consistant en un anneau ou un segment anneau.

Dans un mode de réalisation du procédé selon l'invention, le dispositif implantable est un implant intra-cornéen consistant en un anneau.

La forme d'anneau pourra être découpée extemporanément par le praticien de manière à être adaptée à la pathologie traitée.

Dans un mode de réalisation du procédé selon l'invention, le dispositif implantable est un implant intra-cornéen consistant en un segment anneau.

Au sens de la présente invention, on entend par « segment d'anneau » une structure de forme circulaire incomplète, de moins de 360° en continu.

Dans un mode de réalisation, le procédé fabrication d'un dispositif implantable, ledit dispositif implantable étant un implant intra-cornéen consistant en un segment anneau, est caractérisé en ce qu'au moins une étape x) de découpe comprend la découpe d'au moins un segment anneau d'une longueur d'arc de 90° à 350° en continu.

Dans un mode de réalisation, le procédé fabrication d'un dispositif implantable, ledit dispositif implantable étant un implant intra-cornéen consistant en un segment anneau est caractérisé en ce qu'au moins une étape x) de découpe comprend la découpe d'au moins un segment anneau d'une longueur d'arc de 120° à 325° en continu.

Dans un mode de réalisation, le procédé fabrication d'un dispositif implantable, ledit dispositif implantable étant un implant intra-cornéen consistant en un segment anneau est caractérisé en ce qu'au moins une étape x) de découpe comprend la découpe d'au moins un segment anneau d'une longueur d'arc de 160° à 320° en continu.

Dans un mode de réalisation, le procédé fabrication d'un dispositif implantable, ledit dispositif implantable étant un implant intra-cornéen consistant en un segment anneau est caractérisé en ce qu'au moins une étape x) de découpe comprend la découpe d'au moins un segment anneau d'une longueur d'arc de 210° à 260° en continu.

Dans un mode de réalisation, le procédé fabrication d'un dispositif implantable, ledit dispositif implantable étant un implant intra-cornéen consistant en un segment anneau est caractérisé en ce qu'au moins une étape x) de découpe comprend la découpe d'au moins un segment anneau d'une longueur d'arc de 90°, 120°, 160°, 210°, 260° ou 320° en continu.

Dans un mode de réalisation, le procédé fabrication d'un dispositif implantable, ledit dispositif implantable étant un implant intra-cornéen consistant en un anneau ou un segment anneau est caractérisé en ce qu'au moins une étape x) de découpe comprend la découpe d'au moins un anneau ou un segment anneau de diamètre externe compris dans un intervalle allant de 3 à 10 mm.

Dans un mode de réalisation, le procédé fabrication d'un dispositif implantable, ledit dispositif implantable étant un implant intra-cornéen consistant en un anneau ou un segment anneau est caractérisé en ce qu'au moins une étape x) de découpe comprend la découpe d'au moins un anneau ou un segment anneau de diamètre externe compris dans un intervalle allant de 4 à 7 mm.

Dans un mode de réalisation, le procédé fabrication d'un dispositif implantable, ledit dispositif implantable étant un implant intra-cornéen consistant en un anneau ou un segment anneau est caractérisé en ce qu'au moins une étape x) de découpe comprend la découpe d'au moins un anneau ou un segment anneau de diamètre externe compris dans un intervalle allant de 5 à 6 mm.

Dans un mode de réalisation, le procédé fabrication d'un dispositif implantable, ledit dispositif implantable étant un implant intra-cornéen consistant en un anneau ou un segment anneau est caractérisé en ce qu'au moins une étape x) de découpe comprend la découpe d'au moins un anneau ou un segment anneau d'une épaisseur comprise dans un intervalle allant de 150 µm à 500 µm.

Dans un mode de réalisation, le procédé fabrication d'un dispositif implantable, ledit dispositif implantable étant un implant intra-cornéen consistant en un anneau ou un segment anneau est caractérisé en ce qu'au moins une étape x) de découpe comprend la découpe d'au moins un segment anneau d'une épaisseur croissante dans le sens horaire ou anti-horaire.

Dans un mode de réalisation, le procédé fabrication d'un dispositif implantable selon l'invention, ledit dispositif implantable étant un implant intra-cornéen consistant en un anneau ou un segment anneau, est caractérisé en ce que l'étape de réticulation est réalisée sur un support en forme de dôme.

Dans un mode de réalisation, le support en forme de dôme présente une courbure similaire à celle de la cornée interne.

Dans un mode de réalisation, le support en forme de dôme est tel que décrit dans la demande WO2020/245324 (A1).

Dans un mode de réalisation, le procédé fabrication d'un dispositif implantable selon l'invention, ledit dispositif implantable étant un implant intra-cornéen consistant en un anneau ou un segment anneau, est caractérisé en ce que l'étape de réticulation est réalisée sur un support en forme de dôme, la paroi de cordon ombilical étant disposée de sorte que l'épithélium de la paroi de cordon ombilical est du côté concave.

La présente invention concerne également un implant intra-cornéen consistant en un anneau ou un segment anneau constitué de paroi de cordon ombilical.

Dans un mode de réalisation, l'implant intra-cornéen consistant en un anneau ou un segment anneau selon la présente invention est caractérisé en ce que la paroi de cordon ombilical est réticulé.

Dans le mode de réalisation, la réticulation est telle que précédemment décrite.

Dans un mode de réalisation, l'implant intra-cornéen consistant en un anneau ou un segment anneau selon la présente invention est caractérisé en ce que la paroi de cordon ombilical est traitée avec une base forte pendant une durée d'au moins une heure.

Dans le mode de réalisation, le traitement avec une base forte pendant une durée d'au moins une heure est tel que précédemment décrit.

Dans un mode de réalisation, l'implant intra-cornéen consistant en un anneau ou un segment anneau selon la présente invention est caractérisé en ce que la paroi de cordon ombilical est traitée avec une base forte pendant une durée d'au moins une heure puis réticulée.

Dans un mode de réalisation, l'implant intra-cornéen consistant en un anneau ou un segment anneau selon la présente invention est caractérisé en ce qu'il est viro inactivé.

Dans un mode de réalisation, l'implant intra-cornéen consistant en un anneau ou un segment anneau selon la présente invention est caractérisé en ce qu'il est stérile.

Dans un mode de réalisation, l'implant intra-cornéen consistant en un anneau ou un segment anneau selon la présente invention est caractérisé en ce qu'il est lyophilisé.

Dans un mode de réalisation, l'implant intra-cornéen consistant en un anneau ou un segment anneau selon la présente invention est caractérisé en ce qu'il est dessiqué.

Dans un mode de réalisation, l'implant intra-cornéen consistant en un anneau ou un segment anneau selon la présente invention est caractérisé en ce que la paroi de cordon ombilical a subi au moins une étape de traitement avec une base forte pendant une durée d'au moins une heure et au moins une étape de réticulation, ladite paroi de cordon ombilical n'étant pas traitée par des protéases et/ou des collagénases.

Dans le mode de réalisation, l'implant intra-cornéen consistant en un segment anneau selon la présente invention est caractérisé par une longueur d'arc de 90° à 350° en continu.

Dans le mode de réalisation, l'implant intra-cornéen consistant en un segment anneau selon la présente invention est caractérisé par une longueur d'arc de 120° à 325° en continu.

Dans le mode de réalisation, l'implant intra-cornéen consistant en un segment anneau selon la présente invention est caractérisé par une longueur d'arc de 160° à 320° en continu.

Dans le mode de réalisation, l'implant intra-cornéen consistant en un segment anneau selon la présente invention est caractérisé par une longueur d'arc de 210° à 260° en continu.

Dans le mode de réalisation, l'implant intra-cornéen consistant en un segment anneau selon la présente invention est caractérisé par une longueur d'arc de 90°, 120°, 160°, 210°, 260° ou 320° en continu

Dans un mode de réalisation, l'implant intra-cornéen consistant en un anneau ou un segment anneau selon la présente invention est caractérisé par une épaisseur comprise dans un intervalle allant de 150 µm à 500 µm.

Dans un mode de réalisation, l'implant intra-cornéen consistant en un anneau ou un segment anneau selon la présente invention est caractérisé par une épaisseur croissante dans le sens horaire ou anti-horaire.

Dans le mode de réalisation, l'implant intra-cornéen consistant en un anneau ou un segment anneau selon la présente invention est caractérisé par un diamètre externe de 3 à 12 mm.

Dans le mode de réalisation, l'implant intra-cornéen consistant en un anneau ou un segment anneau selon la présente invention est caractérisé par un diamètre externe de 4 à 7 mm.

Dans le mode de réalisation, l'implant intra-cornéen consistant en un anneau ou un segment anneau selon la présente invention est caractérisé par un diamètre externe de 5 à 6 mm.

La présente invention concerne également un procédé de traitement du kératocône chez un sujet nécessitant un tel traitement, ledit procédé comprenant les étapes suivantes :
a) identifier l'irrégularité de la cornée ;
b) fournir un implant intra-cornéen consistant en un anneau ou un segment anneau selon la présente invention ;
c) préparer le lieu intra cornéen d'épaississement ; et
d) implanter l'implant intra-cornéen consistant en un anneau ou un segment anneau selon la présente invention sur ladite irrégularité.

Le dispositif implantable peut également être une valve cardiaque artificielle.

Dans un mode de réalisation du procédé selon l'invention, le dispositif implantable est valve cardiaque artificielle.

Dans un mode de réalisation du procédé selon l'invention, le dispositif implantable est une valve cardiaque artificielle choisie dans le groupe comprenant : une valve aortique, une valve pulmonaire, une valve mitrale et/ou une valve tricuspide.

Dans un mode de réalisation, le procédé fabrication d'un dispositif implantable, ledit dispositif implantable étant une valve cardiaque artificielle, est caractérisé en ce que l'étape c) de réticulation est précédé d'une étape w') de mise en forme comprenant les sous étapes de :
w'₁) sélection d'un segment tubulaire de paroi de cordon ombilical (1) dont le diamètre est adapté à la valve cardiaque à reproduire, et fermeture (2) en un point non terminal dudit segment tubulaire de paroi de cordon ombilical,
w'₂) insertion d'un support (3) à l'intérieur du segment de paroi de cordon ombilical (1), la zone de fermeture (2) du segment de paroi de cordon (1) étant centrée à l'extrémité du support (3), ledit support (3) comprenant à l'extrémité des empreintes (4) correspondant aux feuillets de la valve cardiaque à reproduire,
w'₃) dépose de masse de marquage (5) à la surface de manière à recréer la forme et/ou la courbure des feuillets de la valve cardiaque à reproduire.

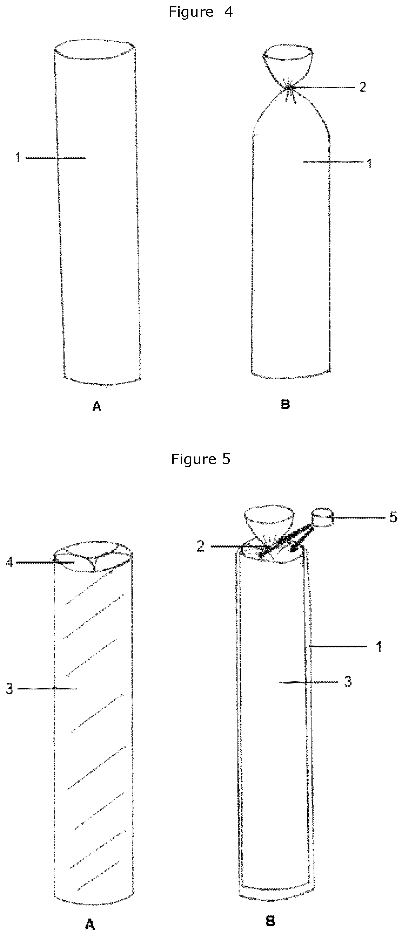

Les sous étapes de l'étape w') de mise en forme sont en particulier représentées aux figures 4 et 5.

Dans un mode de réalisation, l'étape w'₁) de fermeture (2) comprend au moins une étape de nouage, de suture et/ou de collage de la paroi de cordon ombilical.

Dans un mode de réalisation, l'étape w'₁) de fermeture (2) comprend au moins une étape de nouage de la paroi de cordon ombilical dans lequel le nœud est réalisé au moyen d'un fil de suture.

Dans un mode de réalisation, le support inséré à l'étape w'₂) est caractérisé en ce que le support (3) présente un diamètre externe adapté à la taille de la valve cardiaque à reproduire.

Dans un mode de réalisation, l'étape w') de mise en forme comprend en outre une sous étape w'₄) de découpe des feuillets de la valve cardiaque à reproduire.

Alternativement, l'étape de découpe des feuillets de la valve cardiaque à reproduire pourra être réalisée après l'étape de réticulation.

La présente invention concerne également une valve cardiaque artificielle constituée de paroi de cordon ombilical.

Dans un mode de réalisation, la valve cardiaque artificielle selon la présente invention est caractérisée en ce que la paroi de cordon ombilical au niveau des segments de valves est réticulée.

Dans le mode de réalisation, la réticulation est telle que précédemment décrite.

Dans un mode de réalisation, la valve cardiaque artificielle selon la présente invention est caractérisée en ce que la paroi de cordon ombilical sujette à la réticulation est traitée avec une base forte pendant une durée d'au moins une heure.

Dans le mode de réalisation, le traitement avec une base forte pendant une durée d'au moins une heure est tel que précédemment décrit.

Dans un mode de réalisation, la valve cardiaque artificielle selon la présente invention est caractérisée en ce que la paroi de cordon ombilical est traitée avec une base forte pendant une durée d'au moins une heure puis réticulée.

Dans un mode de réalisation, la valve cardiaque artificielle selon la présente invention est caractérisée en ce qu'elle est viro inactivée.

Dans un mode de réalisation, la valve cardiaque artificielle selon la présente invention est caractérisée en ce qu'elle est stérile.

Dans un mode de réalisation, la valve cardiaque artificielle selon la présente invention est caractérisée en ce qu'elle est lyophilisée.

Dans un mode de réalisation, la valve cardiaque artificielle selon la présente invention est caractérisée en ce qu'elle est dessiquée.

Dans un mode de réalisation, la valve cardiaque artificielle selon la présente invention est caractérisée en ce que la paroi de cordon ombilical a subi au moins une étape de traitement avec une base forte pendant une durée d'au moins une heure et au moins une étape de réticulation, ladite paroi de cordon ombilical n'étant pas traitée par des protéases et/ou des collagénases.

Dans un mode de réalisation, la valve cardiaque artificielle selon la présente invention est obtenue par le procédé selon la présente invention.

Dans les exemples, tous les pourcentages sont donnés en poids sauf indication contraire, la température est exprimée en degré Celsius sauf indication contraire, les essais sont réalisés à température ambiante, soit environ 23°C, sauf indication contraire, et la pression est la pression atmosphérique sauf indication contraire.

### EXEMPLES

### Exemple 1 : Procédé de préparation.

### Exemple 1.a. Exemple de procédé de préparation d'un dispositif implantable consistant une paroi de cordon ombilical réticulée.

Un donneur proprement informé et consentant en accord avec les exigences de la Déclaration d'Helsinki offre en don le cordon ombilical issu d'un accouchement. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le cordon ombilical est récupéré le plus tôt possible en salle d'accouchement. Il est placé dans une boite stérile, comprenant une solution de NaCl à +4°C.

Au laboratoire, en salle stérile, la procédure suivante est appliquée :

Le cordon ombilical est découpé dans le sens de la longueur puis ouvert.

Les vaisseaux ombilicaux sont retirés manuellement de manière à ne conserver que la paroi de cordon.

Dans une première étape de découpe, des bandes de 10 à 30 mm de large sont découpées dans le sens de la longueur de la paroi de cordon ombilical.

Les tissus sont congelés à sec avant traitement.

Les bandes sont déposées dans un bain d'eau purifiée à température ambiante pendant environ 3 heures.

Puis, elles sont transférées dans un bain décontaminant composé d'une solution aqueuse d'éthanol 70% v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, la paroi de cordon ombilical est transférée dans un bain composé de peroxyde d'hydrogène à 30% w/v à température ambiante pendant environ 15 minutes.

Puis les bandes de paroi de cordon ombilical sont transférées dans un bain décontaminant composé d'une solution aqueuse de peroxyde d'hydrogène à 3% w/v à température ambiante pendant environ 1 heure.

L'action chimique est neutralisée dans deux bains comprenant de tampon basique dilué autour d'un pH de 8,5. Les bains de neutralisation s'effectuent à température ambiante pendant environ 15 minutes.

Dans une deuxième étape de découpe, les bandes de paroi de cordon ombilical sont découpés en disques homogènes d'environ 10 à 12 mm de diamètre.

Les disques de paroi de cordon ombilical sont traités la soude 1 molaire pendant 19 heures dans des tubes Falcon.

Afin d'assurer un rééquilibrage du pH et pour éliminer au mieux les résidus organiques se détachant du tissu d'intérêt, les structures obtenues sont transférées dans deux bains successifs de tampon PBS à température ambiante pendant environ 15 minutes afin d'assurer son rééquilibrage physiologique.

Puis les disques sont immergés dans une solution de riboflavine à une concentration de 0.1 g/L pendant 7 minutes, les structures obtenues sont disposées sur un moule simulant une cornée. L'ensemble est ensuite soumis à un rayonnement UV-A de 14,4 joules pendant 2 fois 15 minutes.

Afin d'assurer un rééquilibrage du pH et pour éliminer au mieux les résidus organiques se détachant du tissu d'intérêt, la paroi de cordon ombilical est rincée plusieurs fois dans de l'eau stérile de manière à assurer un rééquilibrage physiologique.

La paroi de cordon ombilical réticulée obtenue est soumise à une étape de séchage par dessication dans les conditions suivantes.
- Température de 8,0°C pendant 5 minutes,
- Température de 8,0° pendant 120 minutes,
- Température de 10,0° pendant 30 minutes sous une pression sous vide de 400 µbar,
- Température de 15,0°C pendant 30 minutes sous une pression sous vide de 400 µbar,
- Température de 20,0°C pendant 30 minutes sous une pression sous vide de 400 µbar,
- Température de 25,0°C pendant 30 minutes sous une pression sous vide de 200 µbar,
- Température de 30,0°C pendant 30 minutes sous une pression sous vide de 200 µbar,
- Température de 35,0°C pendant 30 minutes sous une pression sous vide de 200 µbar,
- Fin du cycle par un retour à l'atmosphère normale et à la température ambiante pendant une durée de 5 minutes.

En dehors de la salle stérile, une dernière étape de stérilisation du dispositif implantable consistant une paroi de cordon ombilical réticulée est effectuée par exposition de celle-ci à des rayonnements gamma à 25-32 kGrays.

### Exemple 1.b. Exemple de procédé de préparation d'une lentille pansement consistant une paroi de cordon ombilical réticulée.

Un donneur proprement informé et consentant en accord avec les exigences de la Déclaration d'Helsinki offre en don le cordon ombilical issu d'un accouchement. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le cordon ombilical est récupéré le plus tôt possible en salle d'accouchement. Il est placé dans une boite stérile, comprenant une solution de NaCl à +4°C.

Au laboratoire, en salle stérile, la procédure suivante est appliquée en condition aseptiques :

Le cordon ombilical est ouvert puis découpé avec un dermatome à l'épaisseur de 0,5 mm dans le sens de la longueur.

Les vaisseaux ombilicaux sont retirés manuellement de manière à ne conserver que la paroi de cordon. Dans une première étape de découpe, des bandes de 10 à 12 mm de large sont découpées dans le sens de la longueur de la paroi de cordon ombilical.

Les tissus sont congelés à sec avant le traitement

Dans un premier temps, les bandes sont déposées dans un bain d'eau purifiée à température ambiante pendant environ 3 heures.

Puis, elles sont transférées dans un bain décontaminant composé d'une solution aqueuse d'éthanol 70% v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, les bandes sont transférées dans un bain composé de peroxyde d'hydrogène à 30% w/v à température ambiante pendant environ 15 minutes.

Puis elles sont transférées dans un bain décontaminant composé d'une solution aqueuse de peroxyde d'hydrogène à 3% w/v à température ambiante pendant environ 1 heure.

L'action chimique est neutralisée dans deux bains de tampon basique dilué autour d'un pH de 8,5. Les bains de neutralisation s'effectuent à température ambiante pendant environ 15 minutes.

Dans une deuxième étape de découpe, les bandes de paroi de cordon ombilical sont découpés en forme de disques sensiblement circulaires homogènes d'environ 10 à 12 mm.

Les disques de paroi de cordon ombilical sont traités la soude 1 molaire pendant 19 heures dans des tubes Falcon.

Afin d'assurer un rééquilibrage du pH et pour éliminer au mieux les résidus organiques se détachant du tissu d'intérêt, ils sont transférés dans deux bains successifs de tampon PBS à température ambiante pendant environ 15 minutes afin d'assurer son rééquilibrage physiologique.

Les structures obtenues sont disposées à plat de façon à être totalement immergées sur un support transparent à la lumière UV-A, en forme de dôme contenant une solution de riboflavine à une concentration de 0.1 g/L. L'ensemble est ensuite soumis à un rayonnement UV-A de 14,4 joules pendant deux fois 15 minutes.

Afin d'assurer un rééquilibrage du pH et pour éliminer au mieux les résidus organiques se détachant du tissu d'intérêt, les structures obtenues sont rincées plusieurs fois dans une solution comprenant de l'eau stérile et du dextrane 5% de manière à assurer un rééquilibrage physiologique.

La paroi de cordon ombilical est transférée dans un dernier bain à l'eau purifiée, à température ambiante, pendant 2 fois 15 minutes.

La lentille pansement consistant une paroi de cordon ombilical réticulée obtenue est conservée dans une solution de dextrane.

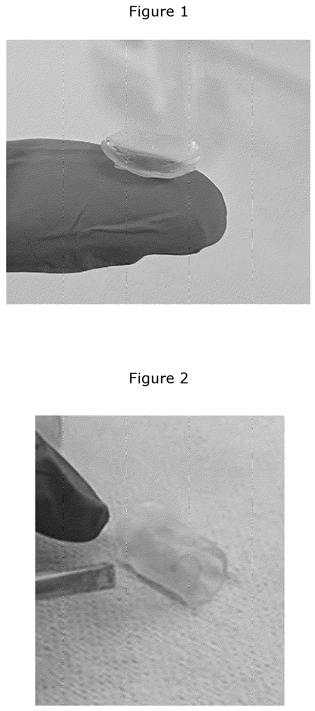

La photographie de la lentille pansement consistant une paroi de cordon ombilical réticulée ainsi obtenue est présentée en figure 1.

### Exemple 1.c. Exemple de procédé de préparation d'un quide de régénération nerveuse, ligamentaire et/ou tendineuse consistant une paroi de cordon ombilical réticulée.

Un donneur proprement informé et consentant en accord avec les exigences de la Déclaration d'Helsinki offre en don le cordon ombilical issu d'un accouchement. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le cordon ombilical est récupéré le plus tôt possible en salle d'accouchement. Il est placé dans une boite stérile, comprenant une solution aqueuse de NaCl à +4°C.

Au laboratoire, en salle stérile, la procédure suivante est appliquée :

Le cordon ombilical est découpé dans le sens de la longueur puis ouvert.

Les vaisseaux ombilicaux sont retirés manuellement de manière à ne conserver que la paroi de cordon. Dans une première étape de découpe, des bandes sont réalisées.

Les bandes sont congelées à -80°C à sec.

Les bandes sont déposées dans un bain d'eau purifiée à température ambiante pendant environ 3 heures.

Puis, elles sont transférées dans un bain décontaminant composé d'une solution aqueuse d'éthanol 70% v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, les bandes sont transférées dans un bain composé de peroxyde d'hydrogène à 30% w/v à température ambiante pendant environ 15 minutes.

Puis les bandes sont transférées dans un bain décontaminant composé d'une solution aqueuse de peroxyde d'hydrogène à 3% w/v à température ambiante pendant environ 1 heure.

L'action chimique appliquée est neutralisée dans deux bains comprenant de tampon basique dilué autour d'un pH de 8,5. Les bains de neutralisation s'effectuent à température ambiante pendant environ 15 minutes.

Les bandes de paroi de cordon ombilical sont traitées à la soude 1 molaire pendant 19 heures dans des tubes Falcon.

Afin d'assurer un rééquilibrage du pH et pour éliminer au mieux les résidus organiques se détachant du tissu d'intérêt, les bandes sont transférées dans deux bains successifs de tampon PBS à température ambiante pendant environ 15 minutes afin d'assurer son rééquilibrage physiologique.

Les bandes sont découpées en rectangles de taille adaptée pour les différentes indications.

Les structures obtenues sont partiellement enroulées autour d'un guide transparent à la lumière UV-A et de taille légèrement supérieure au diamètre souhaité pour le guide du coté membrane amniotique. L'ensemble est immergé dans une solution aqueuse de riboflavine à une concentration de 0.1 g/L de telle sorte que les extrémités de l'ouverture créée lors de l'enroulement partiels ne sont pas immergées.

L'ensemble est ensuite soumis à un rayonnement UV-A de 14,4 joules pendant deux fois 15 minutes.

Afin d'assurer un rééquilibrage du pH et pour éliminer au mieux les résidus organiques se détachant du tissu d'intérêt, les structures obtenues sont rincées plusieurs fois dans une solution comprenant de l'eau stérile et du dextrane 5% de manière à assurer un rééquilibrage physiologique.

Elles sont récupérées et soumises à un séchage par lyophilisation dans les conditions suivantes.
- Température de - 10,0°C pendant 5 minutes,
- Température de - 15,0°C pendant 90 minutes,
- Température de - 45,0°C pendant 5 minutes,
- Température de - 45,0°C pendant 90 minutes,
- Température de 10,0°C pendant 180 minutes sous une pression sous vide de 200 µbar,
- Température de congélation -40°C,
- Mise sous vide à une pression de 300 µbar,
- Température de 10,0°C pendant 210 minutes sous une pression sous vide de 200 µbar,
- Température de 25,0°C pendant 30 minutes sous une pression sous vide de 200 µbar,
- Température de 25,0°C pendant 120 minutes sous une pression sous vide de 200 µbar,
- Température de 35,0°C pendant 60 minutes sous une pression sous vide de 50 µbar,
- Température de 35,0°C pendant 90 minutes sous une pression sous vide de 50 µbar,
- Température de 25,0°C pendant 40 minutes sous une pression sous vide de 50 µbar,
- Température de 25,0°C pendant 20 minutes sous une pression sous vide de 50 µbar,
- Fin du cycle par un retour à l'atmosphère normale et à la température ambiante pendant une durée de 5 minutes.

En dehors de la salle stérile, une dernière étape de stérilisation du guide de régénération nerveuse, ligamentaire et/ou tendineuse consistant une paroi de cordon ombilical réticulée est effectuée par exposition de celle-ci à des rayonnements gamma à 25-32 kGrays.

La photographie du guide de régénération nerveuse, ligamentaire et/ou tendineuse consistant une paroi de cordon ombilical réticulée ainsi obtenu est présentée en figure 2.

### Exemple 1.d. Exemple de procédé de préparation d'un segment anneau formé à partir d'une paroi de cordon ombilical.

Un donneur proprement informé et consentant en accord avec les exigences de la Déclaration d'Helsinki offre en don le cordon ombilical issu d'un accouchement. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le cordon ombilical est récupéré le plus tôt possible en salle d'accouchement. Il est placé dans une boite stérile, comprenant une solution aqueuse de NaCl à +4°C.

Au laboratoire, en salle stérile, la procédure suivante est appliquée :

Le cordon ombilical est découpé dans le sens de la longueur puis ouvert.

La paroi de cordon ombilical est isolée des autres composants du cordon ombilical.

Dans une première étape de découpe, des bandes de 10 à 12 mm de large sont découpées dans le sens de la longueur de la paroi de cordon ombilical.

Elles sont déposées dans un bain d'eau purifiée à température ambiante pendant environ 3 heures.

Puis, elles sont transférées dans un bain décontaminant composé d'une solution aqueuse d'éthanol 70% v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, les bandes de paroi de cordon ombilical sont transférées dans un bain composé de peroxyde d'hydrogène à 30% w/v à température ambiante pendant environ 15 minutes.

Puis elles sont transférées dans un bain décontaminant composé d'une solution aqueuse de peroxyde d'hydrogène à 3% w/v à température ambiante pendant environ 1 heure.

L'action chimique appliquée est neutralisée dans deux bains de tampon basique dilué ayant un pH d'environ 8,5. Les bains de neutralisation s'effectuent à température ambiante pendant environ 15 minutes.

Dans une deuxième étape de découpe, les bandes de paroi de cordon ombilical sont découpés en forme de disques sensiblement circulaires homogènes d'environ 10 à 12 mm.

Les disques de paroi de cordon ombilical sont traités la soude 1 molaire pendant 19 heures dans des tubes Falcon.

Afin d'assurer un rééquilibrage du pH et pour éliminer au mieux les résidus organiques se détachant du tissu d'intérêt, les disques de paroi de cordon ombilical sont transférés dans deux bains successifs de tampon PBS à température ambiante pendant environ 15 minutes afin d'assurer son rééquilibrage physiologique.

Les structures obtenues sont disposées à plat de façon à être totalement immergées sur un support transparent à la lumière UV-A, en forme de dôme d'une courbure similaire à celle d'une cornée interne contenant une solution aqueuse de riboflavine à une concentration de 0.2 g/L. L'ensemble est ensuite soumis à un rayonnement UV-A de 14,4 joules pendant 30 minutes.

Afin d'assurer un rééquilibrage du pH et pour éliminer au mieux les résidus organiques se détachant du tissu d'intérêt, les structures obtenues sont rincées plusieurs fois dans une solution comprenant de l'eau stérile et du dextrane 5% de manière à assurer un rééquilibrage physiologique.

Les structures obtenues sont découpées à l'aide d'un laser femtoseconde en plusieurs segments d'anneaux, avec des longueurs d'arc continues de 160° à 260°, des diamètres variants entre 5 et 6 mm, et des épaisseurs allant de 150 µm à 500 µm.

Les segments d'anneaux sont ensuite soumis à une étape de séchage par dessication dans les conditions suivantes.
- Température de 8,0°C pendant 5 minutes,
- Température de 8,0° pendant 120 minutes,
- Température de 10,0° pendant 30 minutes sous une pression sous vide de 400 µbar,
- Température de 15,0°C pendant 30 minutes sous une pression sous vide de 400 µbar,
- Température de 20,0°C pendant 30 minutes sous une pression sous vide de 400 µbar,
- Température de 25,0°C pendant 30 minutes sous une pression sous vide de 200 µbar,
- Température de 30,0°C pendant 30 minutes sous une pression sous vide de 200 µbar,
- Température de 35,0°C pendant 30 minutes sous une pression sous vide de 200 µbar,
- Fin du cycle par un retour à l'atmosphère normale et à la température ambiante pendant une durée de 5 minutes.

Les structures sont découpées à l'aide d'un scalpel en forme de segments d'anneaux aux différentes dimensions suivantes :
- Longueur d'arc de 120°, 160°, 210°, 260° ou 320° en continu,
- Diamètre externe : 4 à 7 mm,
- Epaisseur 150 µm à 500 µm.

En dehors de la salle stérile, une dernière étape de stérilisation du segment anneau formé à partir d'une paroi de cordon ombilical est effectuée par exposition de celle-ci à des rayonnements gamma à 25-32 kGrays.

### Exemple 1.e. Exemple de procédé de préparation d'une valve cardiaque constituée de paroi de cordon ombilical.

Un donneur informé et consentant en accord avec les exigences de la Déclaration d'Helsinki offre en don le cordon ombilical issu d'un accouchement. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le cordon ombilical est récupéré le plus tôt possible en salle d'accouchement. Il est placé dans une boite stérile, comprenant une solution aqueuse de NaCl à +4°C.

Au laboratoire, en salle stérile, la procédure suivante est appliquée :

Les vaisseaux ombilicaux sont retirés manuellement de manière à ne conserver que la paroi de cordon.

La paroi de cordon ombilical est déposée dans un bain d'eau purifiée à température ambiante pendant environ 3 heures.

Puis, la paroi de cordon ombilical est transférée dans un bain décontaminant composé d'une solution aqueuse d'éthanol 70% v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, la paroi de cordon ombilical est transférée dans un bain composé d'une solution aqueuse de peroxyde d'hydrogène à 30% w/v à température ambiante pendant environ 15 minutes.

Puis la paroi de cordon ombilical est transférée dans un bain décontaminant composé d'une solution aqueuse de peroxyde d'hydrogène à 3% w/v à température ambiante pendant environ 1 heure.

L'action chimique appliquée à la paroi de cordon ombilical est neutralisée dans deux bains comprenant de tampon basique dilué présentant un pH d'environ 8,5. Les bains de neutralisation s'effectuent à température ambiante pendant environ 15 minutes.

La paroi de cordon ombilical est traitée au moyen d'une solution aqueuse de soude 1 molaire pendant 19 heures dans des tubes Falcon.

Afin d'assurer un rééquilibrage du pH et pour éliminer au mieux les résidus organiques se détachant du tissu d'intérêt, la paroi de cordon ombilical est transférée dans deux bains successifs de tampon PBS à température ambiante pendant environ 15 minutes afin d'assurer son rééquilibrage physiologique.

La mise en forme de la paroi de cordon ombilical est schématisée aux figures 4 et 5.

La paroi de cordon traitée (1) est fermée par un nœud (2) au moyen d'un fil de suture en un point central de la longueur de la paroi de cordon ombilical (1).

Un support (3) avec à l'extrémité des empreintes (4) en formes de segment de valve est inséré à l'intérieur de la paroi de cordon ombilical (1) fermée de manière que la zone de fermeture (2) de la paroi de cordon (1) soit centrée à l'extrémité du support (3).

Trois masses de marquage (5) sont déposées sur la surface supérieure fermée de la paroi de cordon (1) en regard des empreintes (4) du support (3) de manière à recréer la forme et la courbure des feuillets d'une valve tricuspide.

La partie valvulaire à former est recouverte d'une solution de riboflavine à une concentration de 0.1 g/L de telle sorte que les extrémités inferieures opposées aux extrémités valvulaires ne soient pas immergés.

Les découpes des segments valvulaires sont réalisées.

L'ensemble est ensuite soumis à un rayonnement UV-A de 14,4 joules pendant 30 minutes.

Afin d'assurer un rééquilibrage du pH et pour éliminer au mieux les résidus organiques se détachant du tissu d'intérêt, la paroi de cordon ombilical réticulée obtenue est rincée plusieurs fois dans une solution comprenant de l'eau stérile et du dextrane 5% de manière à assurer un rééquilibrage physiologique.

Elle est récupérée et soumise à un séchage par lyophilisation dans les conditions suivantes.
- Température de - 10,0°C pendant 5 minutes,
- Température de - 15,0°C pendant 90 minutes,
- Température de - 45,0°C pendant 5 minutes,
- Température de - 45,0°C pendant 90 minutes,
- Température de 10,0°C pendant 180 minutes sous une pression sous vide de 200 µbar,
- Température de congélation -40°C,
- Mise sous vide à une pression de 300 µbar,
- Température de 10,0°C pendant 210 minutes sous une pression sous vide de 200 µbar,
- Température de 25,0°C pendant 30 minutes sous une pression sous vide de 200 µbar,
- Température de 25,0°C pendant 120 minutes sous une pression sous vide de 200 µbar,
- Température de 35,0°C pendant 60 minutes sous une pression sous vide de 50 µbar,
- Température de 35,0°C pendant 90 minutes sous une pression sous vide de 50 µbar.
- Température de 25,0°C pendant 40 minutes sous une pression sous vide de 50 µbar,
- Température de 25,0°C pendant 20 minutes sous une pression sous vide de 50 µbar,
- Fin du cycle par un retour à l'atmosphère normale et à la température ambiante pendant une durée de 5 minutes.

En dehors de la salle stérile, une dernière étape de stérilisation de la valve cardiaque tricuspide artificielle est effectuée par exposition de celle-ci à des rayonnements gamma à 25-32 kGrays.

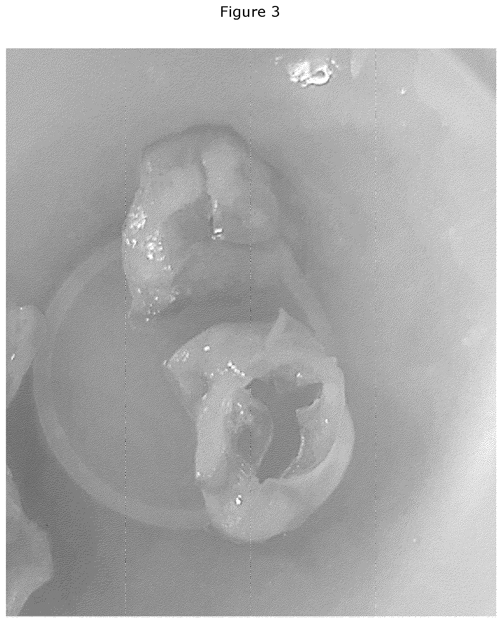

La photographie des valves cardiaques tricuspides artificielles ainsi obtenues est présentée à la figure 3.

### Exemple 1.f. Exemple comparatif de procédé de préparation d'une paroi de cordon ombilical réticulée en absence de traitement avec une base forte.

### Echantillon 1.f.1

Un donneur proprement informé et consentant en accord avec les exigences de la Déclaration d'Helsinki offre en don le cordon ombilical issu d'un accouchement. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le cordon ombilical est récupéré le plus tôt possible en salle d'accouchement. Il est placé dans une boite stérile, comprenant une solution de NaCl à +4°C.

Au laboratoire, en salle stérile, la procédure suivante est appliquée :

Le cordon ombilical est découpé dans le sens de la longueur puis ouvert.

Les vaisseaux ombilicaux sont retirés manuellement de manière à ne conserver que la paroi de cordon.

Dans une première étape de découpe, des bandes de 10 à 30 mm de large sont découpées dans le sens de la longueur de la paroi de cordon ombilical.

Les tissus sont congelés à sec avant traitement.

Les bandes sont déposées dans un bain d'eau purifiée à température ambiante pendant environ 3 heures.

Puis, elles sont transférées dans un bain décontaminant composé d'une solution aqueuse d'éthanol 70% v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, la paroi de cordon ombilical est transférée dans un bain composé de peroxyde d'hydrogène à 30% w/v à température ambiante pendant environ 15 minutes.

Puis les bandes de paroi de cordon ombilical sont transférées dans un bain décontaminant composé d'une solution aqueuse de peroxyde d'hydrogène à 3% w/v à température ambiante pendant environ 1 heure.

L'action chimique est neutralisée dans deux bains comprenant de tampon basique dilué autour d'un pH de 8,5. Les bains de neutralisation s'effectuent à température ambiante pendant environ 15 minutes.

Dans une deuxième étape de découpe, les bandes de paroi de cordon ombilical sont découpés en disques homogènes d'environ 10 à 12 mm de diamètre.

Puis les disques sont immergés dans une solution de riboflavine à une concentration de 0.1 g/L pendant 7 minutes, les structures obtenues sont disposées sur un moule simulant une cornée. L'ensemble est ensuite soumis à un rayonnement UV-A de 14,4 joules pendant 2 fois 15 minutes.

Afin d'assurer un rééquilibrage du pH et pour éliminer au mieux les résidus organiques se détachant du tissu d'intérêt, la paroi de cordon ombilical est rincée plusieurs fois dans de l'eau stérile de manière à assurer un rééquilibrage physiologique.

A l'issu de cette étape, la structure obtenue est entièrement souple, l'étape de réticulation n'ayant produit aucun effet sur la rigidité

La paroi de cordon ombilical obtenue est soumise à une étape de séchage par dessication dans les conditions suivantes.
Température de 8,0°C pendant 5 minutes,
Température de 8,0° pendant 120 minutes,
Température de 10,0° pendant 30 minutes sous une pression sous vide de 400 µbar,
Température de 15,0°C pendant 30 minutes sous une pression sous vide de 400 µbar,
Température de 20,0°C pendant 30 minutes sous une pression sous vide de 400 µbar,
Température de 25,0°C pendant 30 minutes sous une pression sous vide de 200 µbar,
Température de 30,0°C pendant 30 minutes sous une pression sous vide de 200 µbar,
Température de 35,0°C pendant 30 minutes sous une pression sous vide de 200 µbar,
Fin du cycle par un retour à l'atmosphère normale et à la température ambiante pendant une durée de 5 minutes. En dehors de la salle stérile, une dernière étape de stérilisation est effectuée par exposition de celle-ci à des rayonnements gamma à 25-32 kGrays.

A l'issue du procédé, la l'échantillon 1.f.1 est entièrement souple et ne présente aucune résistance lorsqu'il est soumis à une contrainte mécanique. Il se déforme lorsqu'il est maintenu par une de ses extrémités.

### Echantillon 1.f.2

Les disques de paroi obtenues de la même façon que l'échantillon 1.f.1 à l'issue de la deuxième étape de découpe subissent l'ensemble des étapes de traitement décrites à l'exemple 1.a à partir du traitement à la soude 1 molaire pendant 19 heures dans des tubes Falcon.

A l'issu du procédé, l'échantillon 1.f.2 présente une rigidité marquée, ce qui signifie qu'il oppose une résistance notable lorsqu'il est soumis à une contrainte mécanique, tout en restant déformable sans rompre lorsque cette contrainte augmente.

Cette rigidité se manifeste notamment par sa capacité à conserver sa forme initiale, en l'occurrence, une forme plane, même lorsqu'il est maintenu uniquement par l'une de ses extrémités, sans ne se déformer ni fléchir de manière significative.

Cet exemple comparatif démontre donc que l'étape de traitement avec une base forte pendant une durée d'au moins une heure permet d'obtenir les propriétés mécaniques, notamment de rigidité, nécessaires pour les applications visées.

## Revendications

1. Procédé de fabrication d'un dispositif implantable **caractérisé en ce qu'**il comprend les étapes de :
a) On dispose d'une paroi de cordon ombilical,
b) On traite la paroi de cordon ombilical avec une base forte pendant une durée d'au moins une heure,
c) On procède à au moins une étape de réticulation,
d) On obtient un dispositif implantable consistant une paroi de cordon ombilical réticulée,
ledit procédé ne comprenant pas d'étape de traitement de la paroi de cordon ombilical par des protéases et/ou des collagénases.

2. Procédé selon la revendication 1, **caractérisé en ce que** le dispositif implantable est choisi dans le groupe comprenant une lentille biologique, un implant intra-cornéen consistant en un anneau ou un segment anneau, un guide de régénération nerveuse, ligamentaire et/ou tendineuse, et/ou une valve cardiaque artificielle.

3. Procédé selon l'une quelconque des revendications précédentes, dans lequel l'étape b) est **caractérisée en ce que** la base forte est une solution aqueuse d'un sel alcalin choisi dans le groupe comprenant l'hydroxyde de sodium, l'hydroxyde de potassium, l'hydroxyde de calcium, l'hydroxyde de magnésium, l'hydroxyde de lithium, l'hydroxyde d'ammonium, l'hydroxyde de tétraméthylammonium, l'hydroxyde de triméthylbenzylammonium, l'hydroxyde de baryum, l'hydroxyde de strontium.

4. Procédé selon l'une quelconque des revendications précédentes, **caractérisé en ce qu'**il comprend en outre une étape a') de viro-inactivation de la paroi de cordon ombilical suite à l'étape a).

5. Procédé selon l'une quelconque des revendications précédentes, **caractérisé en ce qu'**il comprend en outre une étape b') de neutralisation du pH avant l'étape c) de réticulation.

6. Procédé selon l'une quelconque des revendications précédentes, **caractérisé en ce qu'**il comprend en outre une étape c') de rinçage, consécutive à l'étape c).

7. Procédé selon l'une quelconque des revendications précédentes, **caractérisé en ce qu'**il comprend en outre une étape y) de séchage à la suite de l'étape c), ou alternativement c') lorsque cette étape est mise en œuvre.

8. Procédé selon l'une quelconque des revendications précédentes, **caractérisé en ce qu'**il comprend en outre une étape z) de stérilisation après l'étape c), ou lorsque ces étapes sont mis en œuvre, après l'étape c') ou y).

9. Procédé selon l'une quelconque des revendications précédentes, **caractérisé en ce qu'**il comprend en outre une étape x) de découpe de la paroi de cordon ombilical obtenue à l'issue de l'une quelconque des étapes du procédé.

10. Dispositif implantable obtenu par le procédé selon l'une quelconque des revendications précédentes.

11. Lentille **caractérisé en ce qu'**elle est constituée de paroi de cordon ombilical ayant subi au moins une étape de traitement avec une base forte pendant une durée d'au moins une heure et au moins une étape de réticulation, ladite paroi de cordon ombilical n'étant pas traitée par des protéases et/ou des collagénases.

12. Guide de régénération nerveuse, ligamentaire et/ou tendineuse **caractérisé en ce qu'**il est constitué de paroi de cordon ombilical ayant subi au moins une étape de traitement avec une base forte pendant une durée d'au moins une heure et au moins une étape de réticulation, ladite paroi de cordon ombilical n'étant pas traitée par des protéases et/ou des collagénases.

13. Implant intra-cornéen consistant en un anneau ou un segment anneau **caractérisé en ce qu'**il est constitué de paroi de cordon ombilical.

14. Implant intra-cornéen selon la revendicaiton 13, **caractérisé en ce que** la paroi de cordon ombilical est traitée avec une base forte pendant une durée d'au moins une heure puis réticulée

15. Valve cardiaque artificielle **caractérisé en ce qu'**elle est constituée de paroi de cordon ombilical.
